Europäisches Patentamt
European Patent Office
Office européen des brevets

(19)

(11) Publication number: **0 136 893 B1**

(12) **EUROPEAN PATENT SPECIFICATION**

(45) Date of publication of patent specification: **16.08.89**

(51) Int. Cl.[4]: **A 61 K 31/54,** A 61 K 31/535, C 07 D 279/36, C 07 D 265/34, C 07 F 9/65

(21) Application number: **84306641.6**

(22) Date of filing: **28.09.84**

# (54) Benzo[a]phenothiazines and hydro-derivatives and pharmaceutical compositions containing them.

(30) Priority: **05.10.83 US 539215**

(43) Date of publication of application: **10.04.85 Bulletin 85/15**

(45) Publication of the grant of the patent: **16.08.89 Bulletin 89/33**

(84) Designated Contracting States: **CH DE FR GB IT LI NL**

(56) References cited:
**EP-A-0 115 394**

**CHEMICAL ABSTRACTS, vol. 82, no. 8, 24th February 1975, page 14, no. 51331j, Columbus Ohio (USA). M. GREEN et al.: "Inhibition of DNA polymerases of RNA tumor viruses and cells by rifamycin SV derivatives"**

**JOURNAL OF MEDICINAL CHEMISTRY, vol. 11, no. 3, 26th April 1968, pages 622-623, American Chemical Society: T.G. JACKSON et al.: "Nitrogen mustard derivatives in the phenothiazine and benzophenothiazine series"**

(73) Proprietor: **MERCK FROSST CANADA INC.**
**16711 Trans-Canada Highway**
**Kirkland Quebec (CA)**

(72) Inventor: **Fortin, Rejean**
**4820 Boulevard Leger**
**Montreal Quebec, H1H 1N3 (CA)**
Inventor: **Girard, Yves**
**5498 Shumack**
**Pierresfonds Quebec, H8Z 2Y2 (CA)**
Inventor: **Guindon, Yvan**
**409 Place Closse**
**Ile Bizard Quebec, H9C 1Y7 (CA)**
Inventor: **Lau, Cheuk K.**
**15579 Fernand St.**
**Pieresfonds Quebec, H44 1M6 (CA)**
Inventor: **Rokach, Joshua**
**416 Place Canterbury Chomedey**
**Laval Quebec, H7W 4G9 (CA)**
Inventor: **Yoakim, Christiane**
**5770 Cote St. Luc Road Apt. 12**
**Montreal Quebec, H3X 2E5 (CA)**

(74) Representative: **Crampton, Keith John Allen et al**
**D YOUNG & CO 10 Staple Inn**
**London WC1V 7RD (GB)**

**EP 0 136 893 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:
**CHEMICAL ABSTRACTS, vol. 100, no. 25, 18th June 1984, page 569, no. 209529g, Columbus Ohio (USA);**

## Description

Benzo[a]phenothiazine and hydro-derivatives and analogs thereof are useful as inhibitors of the biosynthesis of mammalian leukotrienes. As such, these compounds are useful therapeutic agents for treating allergic conditions, asthma, cardiovascular disorders, inflammation and pain.

These compounds may be used to treat or prevent mammalian (especially human) disease states such as erosive gastritis; erosive esophagitis; inflammatory bowel disease; ethanol-induced hermorrhagic erosions, hepatic ischemia; noxius agent induced damage or necrosis of hepatic, pancreatic, renal, or myocardial tissue; liver parenchymal damage caused by hepatoxic agents such as $CCl_4$ and D-galoctosamine; ischemic renal failure; disease induced hepatic damage; bile salt induced pancreatic or gastric damage; trauma- or stress-induced cell damage; and glycerol-induced renal failure.

The leukotrienes are a novel group of biologically active substances derived from arachidonic acid through the action of the 5-lipoxygenase enzyme system. There are two groups of leukotrienes derived from a common unstable precursor Leukotriene $A_4$. The first of these are the peptido-lipid leukotrienes, the most important being Leukotrienes $C_4$ and $D_4$. These compounds collectively account for the biologically active material known as the slow reacting substance of anaphylaxis.

The leukotrienes are potent smooth muscle contracting agents, particularly on respiratory smooth muscle but also on other tissues (e.g., gall bladder). In addition, they promote mucous prodution, modulate vascular permeability changes and are potent inflammatory agents in human skin. The most important compound in the second group of leukotriens is Leukotriene $B_4$, a dihydroxy fatty acid. This compound is a potent chemotactic agent for neutrophils and eosinophils. It also effects other cell types such as lymphocytes and for example may modulate the action of T-suppressor cells and natural killer cells. When injected *in vivo,* in addition to promoting the accumulation of leukocytes, Leukotriene $B_4$ is also a potent hyperalgesic agent and can modulate vascular permeability changes through a neutrophil dependent mechanism. See: D. M. Bailey and F. B. Casey, *Ann. Rpts, Med. Chem. 17,* 203 (1982).

As indicated above, the leukotrienes have been implicated in numerous disease states. Inhibition of leukotriene biosynthesis and/or antagonism of leukotriene action, will therefore provide a therapeutic benefit to patients suffering from leukotriene mediated disease states. These disease states include, but are not limited to; asthma, allergic conditions such as allergic rhinitis; skin diseases including psoriasis and atopic dermatitis; inflammation; gouty arthritis; gall bladder spasms, and cardiovascular disorders such as angina.

Certain benzo[a]phenothiazine derivatives of the general Formula A are known compounds:

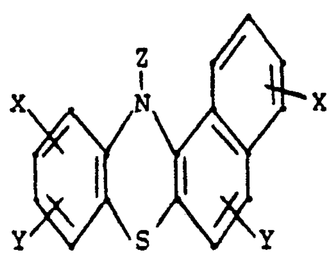

A

Some of these compounds have been used *(inter alia)* as antioxidants, dyes, whitening agents, photosensitizers and polymerization retardants, but although some have been investigated for anti-cancer activity, no significant medical activity has been found for any of them. See for example; T. G. Jackson *et al., J. Org. Chem. 32* 1190—1194 (1967), J. A. Van Allen, *et al., J. Organ. Chem. 34* 1691—1694 (1969), T. G. Jackson *et al. J. Med. Chem. 11* 622—623 (1968), N. L. Agarwal *et al., Acta Chim. Acad. Sci. Hung. 92* 89—97 (1977), R. L. Mital *et al., J. Inst. Chem. (India) 49* 286—287 (1977), J. P. Tiwari *et al., Anorg. Chem., Org. Chem. 33B* 214—215 (1978), J. P. Tiwari *et al., India J. Chem. Sect. B 17B* 408—409 (1079), Y. Ueno *et al., J. Heterocycl. Chem. 19* 167—169 (1982), S. Kikkawa *et al., Chemical Abstracts 76* 139757q (1972) and French Patent No. 1,541,977 (1968).

It has now been discovered that compounds of the Formula A type and their analogues are effective inhibitors of mammalian leukotriene biosynthesis and are thus useful in the treatment of conditions such as asthma, allergies and inflammation in mammals, especially in humans.

In accordance with a first embodiment, the present invention provides a pharmaceutical composition capable of inhibiting leukotriene biosynthesis or action in mammals, especially humans, and containing a pharmaceutically acceptable carrier and a compound that is a pharmaceutically acceptable salt of a compound of Formula I:

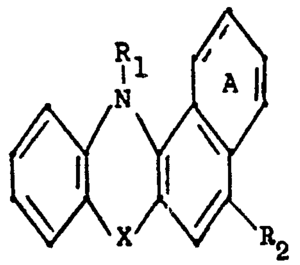

I

in which

X is O, S, SO or $SO_2$;

$R_1$ is H; $C_{1-6}$ alkyl; $C_{1-6}$ acyl; or $COOR_7$ where $R_7$ is ($C_{1-6}$ acyloxy)-($C_{1-6}$ alkyl);

$R_2$ is (a) hydrogen, (b) —$OPO(OR_6)_2$ where

$R_6$ is H, phenyl or $C_{1-6}$ alkyl or (c) $OR_a$ where

$R_a$ is H; $C_{1-5}$ alkyl; $C_{1-5}$ acyl; ($C_{1-6}$ alkoxy)-($C_{1-6}$ alkyl); or $C_{1-4}$ aminoacyl; and the broken lines in ring A indicate that it may be an aromatic or 1,4-dihydroaromatic ring.

Pharmaceutically acceptable salts of the compounds described herein are included within the scope of the present invention. Such salts may be prepared from pharmaceutically acceptable non-toxic bases including inorganic bases and organic bases when the compound is acidic. Salts derived from inorganic bases include sodium, potassium, lithium, ammonium, calcium, magnesium, ferrous, zinc, copper, maganous, aluminum, ferric and manganic salts. Particularly preferred are the potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, tri-methylamine, diethanolamine, diethylamine, triethylamine, tripropylamine ethanolamine, 2-dimethylaminoethanol, 2-diethylaminoethanol, tomethamine, lysine, arginine, histidine, betaine, ethylenediamine, glucosamine, methylglucamine, theobromine, purines, piperazine, N,N'-dibenzylethylenediamine, piperidine, N-ethylpiperidine, morpholine, N-ethylmorpholine, and polyamine resins.

When the compound is basic, salts may be prepared from pharmaceutically acceptable non-toxic acids, including inorganic and organic acids. Such acids include hydrochloric, hydrobromic, sulfuric, nitric, isethionic, methanesulfonic, ethanesulfonic, benzenesulfonic, p-toluenesulfonic, acetic, benzoic, comphorsulfonic, citric, fumaric, gluconic, glutamic, lactic, malic, maleic, mandelic, mucic, pamoic, pantothenic, phosphoric, succinic and tartaric acid. Particularly preferred are hydrochloric, hydrobromic, citric, maleic, phosphoric, sulfuric and tartaric acids.

For helpful discussion of pharmaceutical salts see S. M. Berge *et al.,* Journal of Pharmaceutical Sciences, *66,* 1—19 (1977).

As used herein, the term alkyl, unless otherwise indicated, includes straight chain, branched chain and cycloalkyl groups of the number of carbon atoms shown. The term halogen, as used herein, unless otherwise indicated, includes Cl, Br, I and F.

The present invention also provides a pharmaceutical composition capable of inhibiting leukotriene biosynthesis or action in mammals, especially humans, and containing a pharmaceutically acceptable carrier and a compound having the Formula I or a compound that is a pharmaceutically acceptable salt of a compound of Formula I:

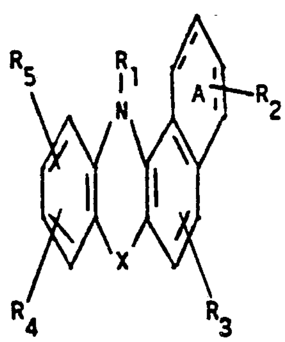

in which the compound has one of the following substituent patterns where, in the table, the number preceding the $R_2$—$R_5$ definitions signifies that group's position on the ring system and the suffix a denotes that the symbol 1 next to the number of a compound indicates which compounds are preferred and the symbol 2 next to the number of a compound indicates which compounds are particularly preferred.

TABLE I

| Compound[a] | X | Ring A | $R_1$ | $R_2$ | $R_3$ | $R_4$ | $R_5$ |
|---|---|---|---|---|---|---|---|
| 1.[1] | S | aromatic | H | 5-OH | H | H | H |
| 2.[1] | S | aromatic | H | 5-$OCOCH_3$ | 6-Cl | H | H |
| 3. | S | aromatic | H | H | H | H | H |
| 4. | S | aromatic | H | 5-OH | 6-Cl | H | H |
| 5. | S | aromatic | H | 5-OH | 6-Cl | 9-Cl | H |
| 6. | S | aromatic | H | 5-OH | 6-Cl | 9-$OCH_3$ | H |
| 7. | S | aromatic | H | 5-$OCOCH_3$ | 6-Cl | 9-$CH_3$ | H |
| 8.[1] | O | aromatic | H | 5-OH | H | H | H |
| 9.[1,2] | O | aromatic | H | 5-$OCOCH_3$ | H | H | H |
| 10. | SO | aromatic | H | 5-OH | H | H | H |
| 11.[1,2] | $SO_2$ | aromatic | H | 5-OH | H | H | H |
| 12. | SO | aromatic | H | H | H | H | H |
| 13. | $SO_2$ | aromatic | H | H | H | H | H |
| 14. | So | aromatic | $COCH_3$ | H | H | H | H |
| 15. | S | aromatic | H | 1-$CO_2H$ | H | H | H |
| 16. | S | aromatic | $CH_3$ | 2-Et | H | H | H |
| 17. | S | aromatic | $COCH_3$ | 2-$COCH_3$ | H | H | H |
| 18. | S | aromatic | H | 2-$COCH_3$ | H | H | H |
| 19. | S | aromatic | H | H | H | H | 10-phenyl |
| 20. | S | aromatic | H | H | H | 8-$CH_3$ | 11-$CH_3$ |
| 21. | S | aromatic | H | H | H | H | 10-$COCH_3$ |
| 22. | S | aromatic | H | 5-OH | 6-$NHCOCH_3$ | 9-F | H |
| 23. | S | aromatic | H | 5-OH | 6-NH-phenyl | 9-Cl | H |
| 24. | SO | aromatic | H | 5-OAc | 6-Cl | H | 10-$CF_3$ |
| 25. | SO | aromatic | H | 5-$OCH_2Ph$ | 6-Cl | H | 10-$CF_3$ |
| 26. | S | aromatic | H | 5-OH | 6-Cl | 8-$CH_3$ | 11-$CH_3$ |
| 27. | S | aromatic | H | 5-$OCH_3$ | 6-Cl | 9-$OCH_3$ | H |
| 28. | $SO_2$ | 1,4-dihydro | H | 5-OH | H | H | H |
| 29. | $SO_2$ | aromatic | $COCH_3$ | 4-$OC_3$ | H | 9-$SCH_3$ | H |
| 30. | O | aromatic | H | H | H | 9-$SO_2CF_3$ | H |

TABLE I (Cont'd)

| Compound[a] | X | Ring A | $R_1$ | $R_2$ | $R_3$ | $R_4$ | $R_5$ |
|---|---|---|---|---|---|---|---|
| 31. | O | aromatic | H | H | H | 11-$CH_3$ | H |
| 32. | S | aromatic | $COCH_3$ | 5-OCOMe | H | H | H |
| 33. | S | aromatic | $CH_2OAc$ | 5-OH | H | H | H |
| 34. | S | aromatic | $CH_2OAc$ | 5-OCOMe | H | H | H |
| 35.[1] | S | aromatic | Me | 5-OH | H | H | H |
| 36.[1,2] | S | aromatic | H | 5-$OCOCH_3$ | H | H | H |
| 37. | S | aromatic | $CH_3$ | 5-$OCH_3$ | H | H | H |
| 38. | S | aromatic | $CH_3$ | 5-$OCOCH_3$ | H | H | H |
| 39. | S | aromatic | $COCH_3$ | 5-OH | H | H | H |
| 40. | S | aromatic | $COCH_3$ | 5-$OCH_3$ | H | H | H |
| 41.[1] | S | aromatic | H | 5-$OCOCH_2NH_2$ | H | H | H |
| 42.[1] | S | 1,4-dihydro | H | 5-OH | H | H | H |
| 43.[1] | S | aromatic | H | 5-$OCOCH(CH_3)_2$ | H | H | H |
| 44.[1] | S | aromatic | H | 5-$OCOC(CH_3)_3$ | H | H | H |
| 45.[1] | S | aromatic | H | 5-$OCOC_6H_5$ | H | H | H |
| 46.[1] | S | aromatic | H | 5-OH | 6-$CH_3$ | H | H |
| 47.[1] | S | aromatic | H | 5-$OCOCH_3$ | 6-$CH_3$ | H | H |
| 48. | S | aromatic | H | 5-OH | 1-OH | 6-$CH_3$ | H |
| 49. | S | aromatic | H | 5-$OCOCH_3$ | 1-OH | 6-$CH_3$ | H |
| 50. | S | aromatic | H | 5-OH | 1-$OCH_3$ | 6-$CH_3$ | H |
| 51. | S | aromatic | H | 5-$OCOCH_3$ | 1-$OCH_3$ | 6-$CH_3$ | H |
| 52. | S | aromatic | H | 5-$OCOC(CH_3)_3$ | 1-$OCH_3$ | 6-$CH_3$ | H |
| 53. | S | 1,4-dihydro | H | 5-$OCOCH_3$ | H | H | H |
| 54. | $SO_2$ | 1,4-dihydro | H | 5-$OCOCH_3$ | H | H | H |
| 55. | SO | aromatic | H | 5-$OCOCH_3$ | H | H | H |
| 56.[1,2] | $SO_2$ | aromatic | H | 5-$OCOCH_3$ | H | H | H |
| 57. | S | aromatic | H | 5-OCH2P$CH_3$ | H | H | H |
| 58. | S | aromatic | H | 5-$OCH_3$ | H | H | H |
| 59. | S | aromatic | H | 5-$OCOC_6H_4$-p-OMe | H | H | H |
| 60. | S | aromatic | H | 5-$OCOC_6H_4$-p-Cl | H | H | H |
| 61. | S | aromatic | H | 5-$OCOC_6H_4$-p-$NH_2$ | H | H | H |

TABLE I (Cont'd)

| Compound[a] | X | Ring A | $R_1$ | $R_2$ | $R_3$ | $R_4$ | $R_5$ |
|---|---|---|---|---|---|---|---|
| 62. | S | aromatic | H | $5\text{-}OCOC_6H_4\text{-}p\text{-}CO_2H$ | H | H | H |
| 63. | S | aromatic | H | $5\text{-}OCOC_6H_4\text{-}O\text{-}CF_3$ | H | H | H |
| 64. | S | aromatic | H | $5\text{-}OCOC_6H_4\text{-}m\text{-}CN$ | H | H | H |
| 65. | S | aromatic | H | $5\text{-}OCO(CH_2)_2\text{-}CO_2H$ | H | H | H |
| 66. | S | aromatic | H | $5\text{-}OCO(CH_2)_4\text{-}CO_2H$ | H | H | H |
| 67. | S | aromatic | H | $5\text{-}OCOCH(NH_2)CH_3$ | H | H | H |
| 68. | S | aromatic | H | $5\text{-}OCOCH(NH_2)\text{-}CH_2C_6H_5$ | H | H | H |
| 69.[1,2] | S | aromatic | $CO_2CHCH_3OAc$ | 5-OH | H | H | H |
| 70.[1,2] | S | aromatic | $CO_2CHCH_3OAc$ | 5-OAc | H | H | H |
| 71. | S | aromatic | $CO_2CHCH_3OAc$ | $5\text{-}OCO_2CHCH_3$<br>\|<br>OAc | H | H | H |
| 72. | S | aromatic | H | $5\text{-}OCO_2CHCH_3$<br>\|<br>OAc | H | H | H |
| 73. | S | aromatic | H | $5\text{-}OPO(OEt)_2$ | H | H | H |
| 74. | S | aromatic | H | 5-OH | 9-OMe | H | H |
| 75. | S | aromatic | H | 5-OAc | 9-OMe | H | H |
| 76.[1] | S | aromatic | H | 5-OH | 9-Me | H | H |
| 77.[1] | S | aromatic | H | 5-OAc | 9-Me | H | H |
| 78. | S | aromatic | H | 5-OH | 9-F | H | H |
| 79. | S | aromatic | H | 5-OAc | 9-F | H | H |
| 80. | S | aromatic | H | 5-OH | 6-OAc | H | H |
| 81. | S | aromatic | H | 5-OAc | 6-OAc | H | H |
| 82. | S | aromatic | Ac | 5-OH | 6-OH | H | H |
| 83. | S | aromatic | Ac | 5-OAc | 6-OAc | H | H |
| 84. | $SO_2$ | aromatic | H | 5-OH | 5-OH | H | H |
| 85.[1,2] | O | aromatic | OAc<br>\|<br>$CO_2CHCH_3$ | 5-OH | H | H | H |
| 86. | $SO_2$ | aromatic | OAc<br>\|<br>$CO_2CHCH_3$ | 5-OH | H | H | H |

The foregoing compounds have unexpected activity as inhibitors of the mammalian biosynthesis of both leukotriene $B_4$, as well as leukotrienes $C_4$, $D_4$, $E_4$ and $F_4$, the active elements of slow reaction substance of anaphylaxis (SRS—A). This inhibition of the biosynthesis of leukotrienes indicates that the compositions would be useful to treat, prevent or ameliorate, in mammals and especially in humans 1) pulmonary conditions including diseases such as asthma, 2) allergies and allergic reactions such as allergic rhinitis,

contact dermatitis or allergic conjuctivitis, 3) inflammation such as arthritis, 4) pain, 5) skin conditions such as psoriasis and 5) cardiovasular conditions such as angina.

Representative compounds of Formula I have been tested using one or more of the following assays to determine their mammalian leukotriene biosynthesis inhibiting activity or their activity in assays relevant to the above disease conditions.

Rat Peritoneal Polymorphonuclear (PMN)
Leukocyte Assay

Rats under ether anesthesia are injected (i.p.) with 8 ml of a suspension of sodium cascinate (6 grams in *ca.* 50 ml water). After 15—24 hr. the rats are sacrificed ($CO_2$) and the cells from the peritoneal cavity are recovered by lavage with 20 ml of buffer (Eagles MEM containing 30 m*M* HEPES adjusted to pH 7.4 with NaOH). The cells are pelleted (350 × g, 5 min.), resuspended in buffer with vigorous shaking, filtered through lens paper, recentrifuged and finally suspended in buffer at a concentration of 10 cells/ml. A 500 µ aliquot of PMN suspension and test compound are preincubated for 2 minutes at 37°C, followed by the addition of 10 µM A—23187. The suspension is stirred for an additional 4 minutes then bioassayed for $LTB_4$ content by adding an aliquot to a second 500 µl portion of the PMN at 37°C. The $LTB_4$ produced in the first incubation causes aggregation of the second PMN, which is measured as a change in light transmission. The size of the assay aliquot is chosen to give a submaximal transmission change (usually −70%) for the untreated control. The percentage inhibition of $LTB_4$ formation is calculated from the ration of transmission change in the sample to the transmission change in the compound-free control.

Asthmatic Rat Assay

Rats were obtained from an inbred line of asthmatic rats. Both female and male rats from 200 to 300 g were used.

Egg albumin (EA), grade V, crystallized and lyophilized, was obtained from Sigma Chemical Co., St. Louis. *Bordetella pertussis* vaccine, containing 30 × $10^9$ killed bacteria per ml was obtained from the Institut Armand-Frappier, Laval des Rapides, Quebec. Aluminum hydroxide was obtained from the Regis Chemical Company, Chicago.

The challenge and subsequent respiratory recordings were carried out in a clear plastic box with internal dimensions 10 × 6 × 4 inches. The top of the box was removable; in use, it was held firmly in place by four clamps and an airtight seal was maintained by a soft rubber gasket. Through the center of each end of the chamber a Devilbiss nebulizer® (No. 40) was inserted via an airtight seal and each end of the box also had an outlet. A Fleisch No. 0000 pneumotachograph® was inserted into one end of the box and coupled to a Grass volumetric pressure transducer® (PT5—A) which was then connected to a Beckman Type R Dynograph® through appropriate couplers. While aerosolizing the antigen, the outlets were open and the pneumotachograph was isolated from the chamber. The outlets were closed and the pneumotachograph and the chamber were connected during the recording or the respiratory patterns. For challenge, 2 ml of a 3% solution of antigen in saline was placed into each nebulizer and the aerosol was generated with air from a small Potter diaphragm pump operating at 10 psi (i.e. 0,7 at) and a flow of 8 liters/minute.

Rats were sensitized by injecting (s.c.) 1 ml of a suspension containing 1 mg EA and 200 mg aluminum hydroxide in saline. Simultaneously, they received an injection (i.p.) of 0.5 ml of *B. pertussis* vaccine. They were used between days 14 and 18 postsensitization. In order to eliminate the serotonin component of the response, rats were pretreated intravenously 5 minutes prior to aerosol challenge with 30 gm $kg^{-1}$ methylsergide. Rats were then exposed to an aerosol of 3% EA in saline for exactly 1 minute, then their respiratory profiles were recorded for a further 25—30 minutes. The duration of continuous dyspnoea was measured from the respiratory recordings.

Compounds were generally administered either intraperitoneally 1 hour prior to challenge or orally 1½ hours prior to challenge. They were either dissolved in dimethylsulfoxide or suspended in 0.1% methylcellulose and 0.5% Polysorbate 80. The volume injected has 2 ml $kg^{-1}$ (intraperitoneally) or 10 ml $kg^{-1}$ (orally). Prior to oral treatment rats were starved overnight. Their activity was determined in terms of their ability to decrease the duration of symptoms of dyspnoea in comparison with a group of vehicle-treated controls. Usually, a compound was evaluated at a series of doses and an $ED_{50}$ was determined. This was defined as the dose (mg/kg) which would inhibit the duration of symptoms by 50%.

PAF-Induced Hyperalgesia Assay

Female Sprague-Dawley rats, 35—40 g were fasted overnight. Platelet activating factor, PAF, (L-lecithin B-acetyl O-alkyl) 1 µg/0.1 ml was given by subplantar injection in the rat paw. The compounds to be evaluated were homogenized in Aqueous Vehicle (0.9% benzyl alcohol, 0.5% Tween® 80 and 0.4% methylcellulose) and administered orally in a volume of 0.1 ml, 30 minutes prior to PAF.

Animals were tested 1, 2, 3 and 4 hours after PAF administration. The vocalization threshold, defined as the pressure (mmHg) needed to evoke a squeak response, was recorded for both the injected and contralateral paw. No animal was subjected to pressure greater than 60 mmHg (8 kPa). Hyperalgesia is defined as a decrease in vocalization threshold as compared to a normal paw. Percent inhibition of hyperalgesia was calculated as the proportion of animals with vocalization thresholds greater than 200% of controls.

Table II gives data obtained from those assays and various compounds of Formula I.

Following in Table II is data obtained using these various assays with representative compounds of Formula I.

TABLE II
Assay Results

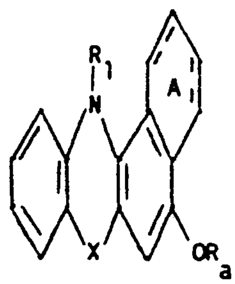

| Ring A | $R_1$ | $R_a$ | X | PMN $IC_{50}$ (mg/ml) | Asth. Rat % Inhibition and Dose (mg/kg) (p.o.) | PAF Induced Hyperalgesia % Inhibition and Dose (mg/kg) (p.o.) |
|---|---|---|---|---|---|---|
| aromatic | H | Ac | S | 0.025—0.05 | $ED_{50}$: 4 mg/kg | $ED_{50}$: 0.145 mg/kg |
| aromatic | Me | Me | S | 5 | — | 80% (3)<br>40% (1) |
| aromatic | Me | Ac | S | 0.05 | — | 30—60% (1) |
| aromatic | Ac | H | S | 1—5 | — | — |
| aromatic | Ac | Me | S | 5 | — | — |
| aromatic | H | H | S | 0.02—0.1 | 54% (1.5) | — |
| aromatic | H | $COCH_2NH_2$<br>·HCl | S | 0.04—0.2 | 44% (3) | —<br>21% (1.5) |
| 1,4-dihydro | H | H | S | 0.04 | — | — |
| aromatic | H | $CH_2OCH_3$ | S | 1—5 | — | — |
| aromatic | H | COPh | S | 0.5 | 18% (5) | — |
| aromatic | H | $COC(CH_3)_3$ | S | 0.1—1 | 54% (5) | — |
| aromatic | H | $OR_a$=H | S | 0.2—1 | — | — |
| aromatic | H | Me | S | 0.1 | — | — |
| aromatic | H | $PO(OEt)_2$ | S | 0.2—1 | — | — |
| aromatic | Ac | Ac | S | — | 47% (5) | — |
| aromatic | $CO_2CHCH_3$<br>OAc | H | S | 0.2—1 | 49% (1.5) | 60% (3) |
| aromatic | H | H | $SO_2$ | 0.2—1 | 67% (5) | — |
| aromatic | H | Ac | $SO_2$ | 0.2—1 | 42% (5) | — |

TABLE II (cont'd.)

| Ring A | $R_1$ | $R_a$ | X | PMN $IC_{50}$ (mg/ml) | Asth. Rat % Inhibition and Dose (mg/kg) (p.o.) | PAF Induced Hyperalgesia % Inhibition and Dose (mg/kg) (p.o.) |
|---|---|---|---|---|---|---|
| aromatic | H | Ac | SO | 0.2—1 | — | — |
| aromatic | H | Ac | O | 0.05—0.5 | 44% (5) | — |

The pharmaceutical compositions will contain a sufficient amount of a compound of Formula I in a dosage form suitable for inhibiting the mammalian biosynthesis of leukotrienes or, for the treatment desired. The effective concentration of Formula I compound in the composition will vary as required by the severity of the condition to be treated, the particular compound of Formula I, the mode of administration, dosage form and pharmacological effect and level desired. A general daily dosage of Formula I (for uses other than cytoprotection) will range from 100 µg to 200 mg/kg of body weight. A preferred daily dosage range is from 1 to 100 mg/kg, particularly 2 to 50 mg/kg.

For treating pulmonary conditions such as asthma, the mode of administration may be oral, parenteral, by inhalation or by suppository. Suitable oral dosage forms are tablets, elixirs, emulsions, solutions and capsules, including delayed or sustained-release capsules. Parenteral dosage forms include solutions and emulsions. Dosage forms for administration by inhalation include sprays and aerosols. These inhalation formulations may be administered in metered doses ranging from 0.1 to 200 µg, administered as needed.

For treating allergies or allergic reactions such as allergic conjunctivitis and allergic rhinitis, the Formula I compound may be administered by any conventional mode, e.g. orally, parenterally, topically, subcutaneously or by inhalation.

The oral and parenteral dosage forms are the same type as for the pulmonary treatment. The topical application dosage forms include ointments, salves, controlled-release patches, emulsions, solutions, thioxotropic formulations, powders and sprays. For topical application, the amount by weight of the active ingredient (Formula I compound) may vary from 0.001 to 10%.

For treating inflammation the mode of administration may be oral, parenteral or by suppository. The various dosage forms are the same as those described above.

For treating skin diseases such as psoriasis and atopic dermatitis, oral, topical or parenteral administration is useful. For topical application to the diseased area salves, patches, controlled release patches and emulsions are convenient dosage forms.

For use as an analgesic, i.e. for treating pain, any suitable mode of administration may be used, e.g., oral, parenteral, by insufflation or by suppository.

For treating cardiovascular conditions such as angina pectoris, any suitable mode of administration, e.g. oral, parenteral, topical or insufflation, and any suitable dosage form, e.g. pills, liquid formulations, controlled-release capsules or controlled-release skin patches, may be used.

In addition to the common dosage forms set out above, the compound of Formula I may also be administered for the various utilities and indications or for inhibiting leukotriene synthesis by controlled-release means and/or delivery devices such as those described in the following U.S. Patents: US—A—3,845,770, 3,916,899, 3,536,809, 3,598,123, 3,630,200 and 4,008,719. Dosage forms for application to treat the eye are also disclosed in US—A4,348,398.

In preparing suitable dosage forms, conventional compounding procedures and ingredients e.g. diluents and carriers, may be used.

Two assays can be used to measure cytoprotective ability. These assays are; (A) an ethanol-induced gastric ulcer assay and (B) an indomethacin-induced ulcer assay.

A. Ethanol-Induced Gastric Ulcer Assay

Twenty-four-hour-fasted Sprague-Dawley (S.D.) rats are perorally (p.o.) dosed with 1.0 ml absolute ethanol. Fifteen minutes prior to ethanol administration, groups of rats each receive either an aqueous vehicle (aqueous methylcellulose 5% wt.) or the test compound at various doses perorally. One hour later, the animals are sacrificed and stomach mucosae are examined for resulting lesions.

B. Indomethacin-Induced Ulcer Assay

Indomethacin, 10 kg/kg p.o., is used to induce ulcers in 24-hour-fasted S.D. rats. Fifteen minutes prior to indomethacin administration, groups of rats each receive either an aqueous vehicle (5% by weight methylcellulose) or the test compound at various doses perorally. Four hours later the animals are sacrificed and stomach mucosae are examined for resulting ulcers.

As cytoprotective agents, the compounds of Formula I may generally be administered at a dosage range of 0.02 mg/kg to 100 mg/kg of body weight. The exact amount of inhibitor to be used will depend on, *inter alia*, whether it is being administered to heal damaged cells or to avoid future damage, on the nature of the damaged cells (e.g., gastro-intestinal ulcerations vs. nephrotic necrosis), and on the nature of the causative agent. An example of the use of a compound of Formula I in avoiding future damage would be co-administration of a compound of the formula I with a non-steroidal antiinflammatory drug that might otherwise cause such damage (for example, indomethacin). For such use, the compound of Formula I is administered from 30 minutes prior up to 30 minutes after administration of the NSAID. Preferably, it is administered prior to or simultaneously with the NSAID (for example, in a combination dosage form).

The effective daily dosage level for compounds of Formulae I inducing cytoprotection in mammals, especially humans, will range from 0.02 mg/kg to 100 mg/kg, preferably from 0.02 mg/kg to 30 mg/kg. The dosage may be administered in single or divided individual doses, using various dosage forms, as described above.

The pharmaceutical compositions of the present invention comprise a compound of formula I as an active ingredient or a pharmaceutically acceptable salt thereof, and may also contain a pharmaceutically acceptable carrier and optionally other therapeutic ingredients. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray presentation from pressurized packs or a nebuliser. The preferred composition for inhalation is a powder which may be formulated as a cartridge from which the powder composition may be inhaled with the aid of a suitable device. In the case of a pressurized aerosol, the dosage unit may be determined by providing a valve to deliver a metered amount.

In practical use, leukotriene inhibitors of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or intravenous. In preparing the compositions for oral dosage form, any of the usual pharmaceutical media may be used, for example, water, glycols, oils, alcohols, flavoring agents, preservatives and coloring agents in the case of oral liquid preparations, such as suspensions, elixirs and solutions; or carriers such as starches, sugars, diluents, granulating agents, lubricants, binders or and disintegrating agents in the case or oral solid preparations such as powders, capsules and tablets. Because of their ease of administration, tablets and capsules represent the most advantageous oral dosage unit form, in which case solid pharmaceutical carriers are obviously used. If desired, tablets may be sugar-coated or enteric-coated by standard techniques.

In addition to the common dosage forms set out above, the compounds of Formula I may also be administered by controlled-release means and/or delivery devices such as those described in the following U.S. Patents: US—A—3,845,770; 3,916,899, 3,536,809; 3,598,123; 3,630,200 and 4,008,719.

Pharmaceutical compositions of the present invention suitable for oral administration and by inhalation in the case of asthma therapy may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier, which contains one or more ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, lubricating, surface active or dispersing agent. Molded tablets may be made by molding in a suitable machine, a mixture of the powdered compound moistened with an inert liquid diluent. Desirably, each tablet contains from 25 mg to 500 mg of the active ingredient and each cachet or capsule contains from 25 to about 500 mg of the active ingredient.

The following are examples of representative pharmaceutical dosage forms for the compounds of Formula I:

| Injectable Suspension | mg/ml |
|---|---|
| Compound of Formula I | 2.0 |
| Methylcellulose | 5.0 |
| Tween® 80 | 0.5 |
| Benzyl alcohol | 9.0 |
| Methyl paraben | 1.8 |
| Propyl paraben | 0.2 |
| Water for injection to a total volume of 1 ml | |

| Tablet | mg/tablet |
|---|---|
| Compound of Formula I | 25.0 |
| Microcrystalline Cellulose | 325.0 |
| Providone | 14.0 |
| Microcrystalline Cellulose | 90.0 |
| Pregelatinized Starch | 43.5 |
| Magnesium Stearate | 2.5 |
| | 500 |

| Capsule | mg/capsule |
|---|---|
| Compound of Formula I | 25 |
| Lactose Powder | 573.5 |
| Magnesium Stearate | 1.5 |
| | 600 |

In addition to the compounds of Formula I, the pharmaceutical compositions of the present invention can also contain other active ingredients, such as cyclooxygenase inhibitors, non-steroidal anti-inflammatory drugs (NSAIDs), peripheral analgesic agents such as zomepirac or diflunisal. The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from 1000:1 to 1:1000, preferably 200:1 to 1:200. Combinations of a compound of the Formula I and other active ingredients will generally also be within the aforementioned range, but in each case, an effective dose of each active ingredient should be used.

NSAIDs can be characterized into five groups:

(1) the propionic acid derivatives;

(2) the acetic acid derivatives;

(3) the fenamic acid derivatives;

(4) the biphenylcarboxylic acid derivatives; and

(5) the oxicams

or a pharmaceutically acceptable salt thereof.

The propionic acid derivatives which may be used comprise: ibuprofen, ibuprufen aluminum, indoprofen, ketoprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Structurally related propionic acid derivatives having similar analgesic and anti-inflammatory properties are also intended to be included in this group.

Thus, "propionic acid derivatives" as defined herein are non-narcotic analgesics/non-steroidal anti-inflammatory drugs having a free $-CH(CH_3)COOH$ or $-CH_2CH_2COOH$ group (which optionally can be in

the form of a pharmaceutically acceptable salt group, e.g., $—CH(CH_3)COO^-Na^+$ or $—CH_2CH_2COO^-Na^+$), typically attached directly or via a carbonyl function to a ring system, preferably to an aromatic ring system.

The acetic acid derivatives which may be used comprise: indomethacin, which is a preferred NSAID, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structurally related acetic acid derivatives having similar analgesic and antiinflammatory properties are also intended to be encompassed by this group.

Thus, "acetic acid derivatives" as defined herein are non-narcotic analgesics/non-steroidal antiinflammatory drugs having a free $—CH_2COOH$ group (which optionally can be in the form of a pharmaceutically acceptable salt group, e.g. $—CH_2COO^-Na^+$), typically attached directly to a ring system, preferably to an aromatic or heteroaromatic ring system.

The fenamic acid derivatives which may be used comprise: mefenamic acid, meclofenamic acid, flufenamic acid, niflumic acid and tolfenamic acid. Structurally related fenamic acid derivatives having similar analgesic and anti-inflammatory properties are also intended to be encompassed by this group.

Thus, "fenamic acid derivatives" as defined herein are non-naccotic analgesics/non-steroidal anti-inflammatory drugs which contain the basic structure:

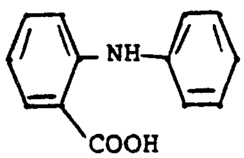

which can bear a variety of substituents and in which the free —COOH group can be in the form of a pharmaceutically acceptable salt group, e.g., $—COO^-Na^+$.

The biphenylcarboxylic acid derivatives which can be used comprise: diflunisal and flufenisal. Structurally related biphenylcarboxylic acid derivatives having similar analgesic and anti-inflammatory properties are also intended to be encompassed by this group.

Thus, "biphenylcarboxylic acid derivatives" as defined herein are non-narcotic analgesics/non-steroidal anti-inflammatory drugs which contain the basic structure:

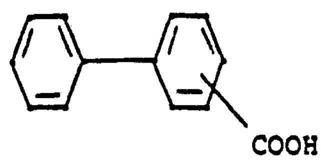

which can bear a variety of substituents and in which the free —COOH group can be in the form of a pharmaceutically acceptable salt group, e.g., $—COO^-Na^+$.

The oxicams which can be used in the present invention comprise: piroxicam, sudoxicam, isoxicam and 4-hydroxyl-1,2-benzothiazine 1,2-dioxide 4-(N-phenyl)-carboxamide. Structurally related oxicams having similar analgesic and anti-inflammatory properties are also intended to be encompassed by this group.

Thus, "oxicams" as defined herein are non-narcotic analgesics/non-steroidal anti-inflammatory drugs which have the general formula:

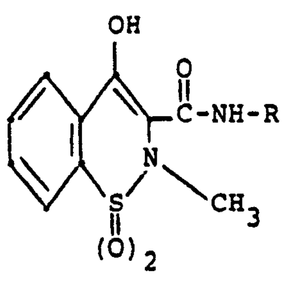

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole,

loxoprofen, lysin clonixinate, meclofenamate sodium, meseclazone, miroprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acid, tiaramide HCl, tiflamizole, timegadine, tioxaprofen, tolfenamic acid, tolpadol, tryptamid, ufenamate, and zidometacin.

The following NSAIDs may also be used:

2-[4-(2-thiazolyloxy)-phenyl]-propionic acid
2,3,5-trimethyl-6-(12-hydroxy-5,10-dodecadiynyl-1,4-benzoquinone]
2-(8-methyl-10,11-dihydro-11-oxodibenz[b,f]oxepin-2-yl)propionic acid
3,4,5-trimethoxybenzoyl-ibuprofen
guaiphenesin ibuprofenate
[6-[[1-(3,4-dihydro-8-hydroxy-1-oxo-1H-2-benzopyran-3-yl)-3-methylbutyl]amino]-5-dihydroxy-6-oxo-3-hexanamido]ammonium chloride
chlorhexidine dinaproxenate
4'-acetamidophenyl-2-(5'-p-toluyl-1'-methyl-pyrrole)-acetate
(±)-5-benzoyl-2,3-dihydro-1H-pyrrolizine-1-carboxylic acid tromethamine salt
3-methylamino-1-[3-(trifluoromethyl)-phenyl]-2-pyrazoline
Chinoin-127, a rimazolium analogue
2-(p-methylallylaminophenyl)propionic acid
(±)-α-[[(2-hydroxy-1,1-dimethylethyl)amino]methyl]benzyl alcohol)
5-[5-(4-chlorophenyl)-2-furanyl]-dihydro-2(3H)-furanone
mono(2-ammonium-2-hydroxymethyl-3,3-diol)(2R-cis)-(3-methyloxiranyl)phosphonate
diflunisal lysine salt
3,5-di-t-butyl-4-hydrobenzylidene-γ-butyrolactone
imidazole 2-hydroxybenzoate
methyl 5,6-E-5,6-methanoeicosa-7E,9E,11Z,14E-eicosatetranoate
2,4-diamino-7-methyl-pyrazolo(1,5-α)-1,3,5-triazine
2-(2',4'-difluoro-4-biphenyl)oxypropionic acid
3-methyl-3-(4-acetyl-aminophenoxy)-2,4-dioxobenzocyclo-1-esanone
4-(p-chlorophenyl)-1-(p-fluorophenyl)pyrazole-3-acetic acid
2-aminomethyl-4-t-butyl-6-propionylphenol hydrochloride
glycolic acid [o-(2,6-dichloroanilino)phenyl]acetate ester
the 3-hydroxyphthalide ester of (±)-1-(p-chlorobenzoyl)-5-methoxy-2-methylindole-3-acetic acid
the phthalidyl ester of diclofenac
2,6-di-t-butyl-4-(2'-thenoyl)phenol
2-[4-(2-oxocyclohexylidenemethyl)phenyl]propionic acid
2-(4-biphenylyl)-4-hexenoic acid
11β,17,21-trihydroxy-6α-methylpregna-1,4-diene-3,20-dione 21-acetate 17-propionate
4-(2-bromo-4,5-dimethoxyphenyl)-octahydro-2H-quinolizin-2-one
N-(2-pyridyl)-2-methyl-4-cinnamoyloxy-2H-1,2-benzothiazine-3-carboxamide-1,1-dioxide
17α-ethylthio-9α-fluoro-11β-hydroxy-17β-methylthio-androsta-1,4-diene-3-one
1,1,3-trimethyl-5-phenylbiuret
1-(4-chlorbenzoyl-5-methoxy-2-methyl-1H-indole-3-acetic acid butanediol ester
2-(4-(3-methyl-2-butenyl)phenyl)propionic acid
3-ethyl-1-(3-nitrophenyl)-2,4-[1H,3H]-quinazolindione
6,9-de-epoxy-6,9-(phenylimino)-delta(6,8)-PGI1
1-benzoyl-5-methoxy-2-methylindole-3-acetic acid
(5H-dibenzo[a,d]cyclohepten-5-ylidene)acetic acid

Finally, NSAIDs which may also be used include the salicylates, specifically acetylsalicylic acid, and the phenylbutazones, and pharmaceutically acceptable salts thereof.

Pharmaceutical compositions comprising the Formula I compounds may also contain other inhibitors of the biosynthesis of the leukotrienes as are disclosed in pending European Patent Specifications EP—A—0,138,481, 0,115,394 and 0,140,709.

The compounds of the Formula I may also be used in combination with leukotriene antagonists such as those disclosed in European Patent Specifications EP—A—0,106,565, 0,104,885, 0,056,172 and 0,061,800; and in U.K. Patent Specification GB—A—2,058,785.

Pharmaceutical compositions comprising the Formula I compounds may also contain as the second active ingredient, antihistaminic agents such as benadryl, dramamine, histadyl or phenergan. Alternatively, they may include prostaglandin antagonists such as those disclosed in European Patent Application EP—A—0,011,067 or thromboxane antagonists such as those disclosed in US Patent Specification US—A—4,237,160. They may also contain histidine decarboxyase inhibitors such as α-fluoromethylhistidine, described in US—A—4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in EP—A—0,040,696, and U.S. Patent Specifications

US—A—4,283,408; 4,362,736 and 4,394,508. The pharmaceutical compositions may also contain a $K^+/H^+$ ATPase inhibitor such as omeprazole, disclosed in US—A—4,255,431.

Another embodiment of the present invention are the novel compounds and their pharmaceutically acceptable salts, encompassed by Formula I. These compounds are described in Table III. The number preceding the $R_2$—$R_5$ definitions signifies that group's position on the ring system.

Pharmaceutical compositions comprising the Formula I compounds may also contain as the second active ingredient, antihistaminic agents such as benadryl, dramamine, histadyl or phenergan. Alternatively, they may include prostaglandin antagonists such as those disclosed in EP—A—11,067 or thromboxane antagonists such as those disclosed in US—A—4,237,160. They may also contain histidine decarboxyase inhibitors such as α-fluoromethylhistidine, described in US—A—4,325,961. The compounds of the Formula I may also be advantageously combined with an $H_1$ or $H_2$-receptor antagonist, such as for instance cimetidine, ranitidine, terfenadine, famotidine, aminothiadiazoles disclosed in EP—application nr. 81102976.8 and like compounds, such as those disclosed in U.S. Patent Nos. i.e. US—A—4,283,408; —4,362,736; —4,394,508 and EP—A—40,696. The pharmaceutical compositions may also contain a $K^+/H^+$ ATPhase inhibitor such as omeprazole, disclosed in U.S. Pat., i.e. US—A—4,255,431. Each of the references referred to in this paragraph is hereby incorporated herein by reference.

Another embodiment of the present invention are the novel compounds and their pharmaceutically acceptable salts, encompassed by Formula I. These compounds are described in Table III. The number preceding the $R_2$—$R_5$ definitions signifies that group's position on the ring system.

TABLE III

Novel Compounds of Formula I

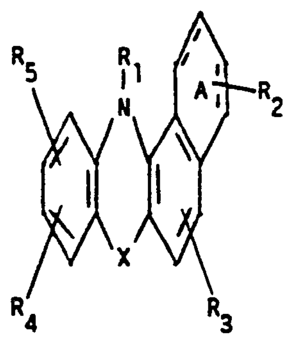

| Compound[a] | X | Ring A | $R_1$ | $R_2$ | $R_3$ | $R_4$ | $R_5$ |
|---|---|---|---|---|---|---|---|
| 1.[1] | S | aromatic | H | 5-$OCOCH_3$ | H | H | H |
| 2. | S | aromatic | $CH_3$ | 5-$OCH_3$ | H | H | H |
| 3. | S | aromatic | $CH_3$ | 5-$OCOCH_3$ | H | H | H |
| 4. | S | aromatic | $COCH_3$ | 5-OH | H | H | H |
| 5. | S | aromatic | $COCH_3$ | 5-$OCH_3$ | H | H | H |
| 6. | S | aromatic | H | 5-$OCOCH_2NH_2$ | H | H | H |
| 7. | S | 1,4-dihydro | H | 5-OH | H | H | H |
| 8. | S | aromatic | H | 5-$OCOCH(CH_3)_2$ | H | H | H |
| 9. | S | aromatic | H | 5-$OCOC(CH_3)_3$ | H | H | H |
| 10. | S | aromatic | H | 5-$OCOC_6H_5$ | H | H | H |
| 11. | S | aromatic | H | 5-OH | 6-$CH_3$ | H | H |
| 12. | S | aromatic | H | 5-$OCOCH_3$ | 6-$CH_3$ | H | H |
| 13. | S | aromatic | H | 5-OH | 1-OH | 6-$CH_3$ | H |

TABLE III (cont'd)

| Compound[a] | X | Ring A | $R_1$ | $R_2$ | $R_3$ | $R_4$ | $R_5$ |
|---|---|---|---|---|---|---|---|
| 14. | S | aromatic | H | 5-$OCOCH_3$ | 1-OH | 6-$CH_3$ | H |
| 15. | S | aromatic | H | 5-OH | 1-$OCH_3$ | 6-$CH_3$ | H |
| 16. | S | aromatic | H | 5-$OCOCH_3$ | 1-$OCH_3$ | 6-$CH_3$ | H |
| 17. | S | aromatic | H | 5-$OCOC(CH_3)_3$ | 1-$OCH_3$ | 6-$CH_3$ | H |
| 18. | S | 1,4-dihydro | H | 5-$OCOCH_3$ | H | H | H |
| 19. | SO | aromatic | H | 5-$OCOCH_3$ | H | H | H |
| 20.[1] | $SO_2$ | aromatic | H | 5-$OCOCH_3$ | H | H | H |
| 21. | SO | aromatic | H | 5-OH | H | H | H |
| 22.[1] | $SO_2$ | aromatic | H | 5-OH | H | H | H |
| 23. | S | aromatic | H | 5-$OCH_2OCH_3$ | H | H | H |
| 24. | S | aromatic | H | 5-$OCH_3$ | H | H | H |
| 25. | S | aromatic | $COCH_3$ | 5-$OCOCH_3$ | H | H | H |
| 26. | S | aromatic | $CH_3$ | 5-OH | H | H | H |
| 27.[1] | S | aromatic | $CO_2CHCH_3$<br>OAc | 5-OH | H | H | H |
| 28.[1] | S | aromatic | $CO_2CHCH_3$<br>OAc | 5-OAc | H | H | H |
| 29. | S | aromatic | $CH_2CHCH_3$<br>OAc | 5-$OCO_2CHCH_3$<br>\|<br>OAc | H | H | H |
| 30. | S | aromatic | H | 5-$OCO_2CHCH_3$<br>\|<br>OAc | H | H | H |
| 31. | S | aromatic | H | 5-$OPO(OEt)_2$ | H | H | H |
| 32. | S | aromatic | H | 5-OH | 9-OMe | H | H |
| 33. | S | aromatic | H | 5-OAc | 9-OMe | H | H |
| 34. | S | aromatic | H | 5-OH | 9-Me | H | H |
| 35. | S | aromatic | H | 5-OAc | 9-Me | H | H |
| 36. | S | aromatic | H | 5-OH | 9-F | H | H |
| 37. | S | aromatic | H | 5-OAc | 9-F | H | H |
| 38. | S | aromatic | H | 5-OH | 6-OAc | H | H |
| 39. | S | aromatic | H | 5-OAc | 6-OAc | H | H |
| 40. | S | aromatic | Ac | 5-OH | 6-OH | H | H |

TABLE III (cont'd)

| Compound[a] | X | Ring A | $R_1$ | $R_2$ | $R_3$ | $R_4$ | $R_5$ |
|---|---|---|---|---|---|---|---|
| 41. | S | aromatic | Ac | 5-OAc | 6-OAc | H | H |
| 42. | $SO_2$ | aromatic | H | 5-OH | 6-OH | H | H |
| 43. | O | aromatic | H | 5-OH | H | H | H |
| 44.[1] | O | aromatic | H | 5-OAc | H | H | H |
| 45.[1] | O | aromatic | $CO_2CH(OAc)CH_3$ | 5-OH | H | H | H |
| 46. | $SO_2$ | aromatic | $CO_2CH(OAc)CH_3$ | 5-OH | H | H | H |
| 47. | S | aromatic | H | 5-OH | 9-$OCH_2CO_2H$ | H | H |
| 48. | S | aromatic | H | 5-OH | 6-Br | 9-$CH_2CO_2H$ | H |
| 49.[1] | O | aromatic | $CO_2CH(OAc)CH_3$ | 5-OH | H | H | H |

[a] The symbol 1 next to the number of a compound indicates which compounds are preferred.

The compounds of Formula I may be prepared by any process available to the skilled artisan. Several such processes are illustrated in Schemes I to IV below.

SCHEME I

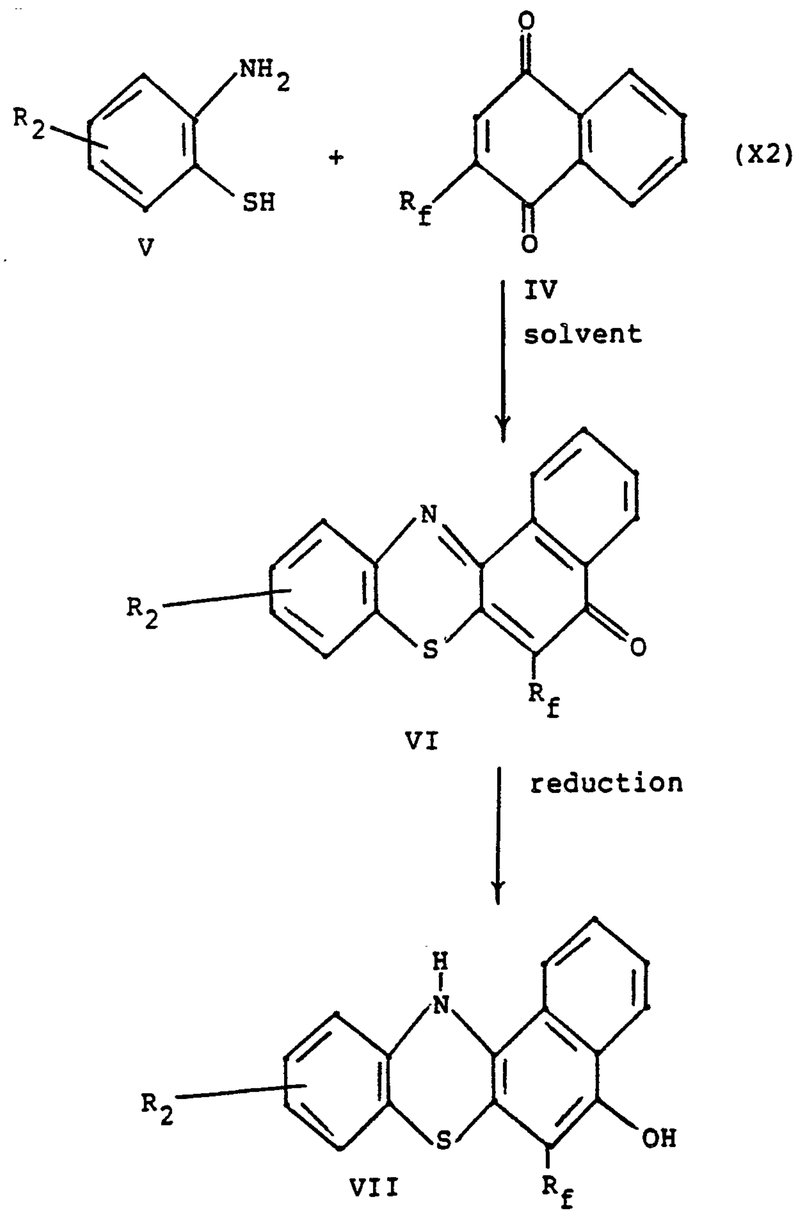

SCHEME I (Continued)

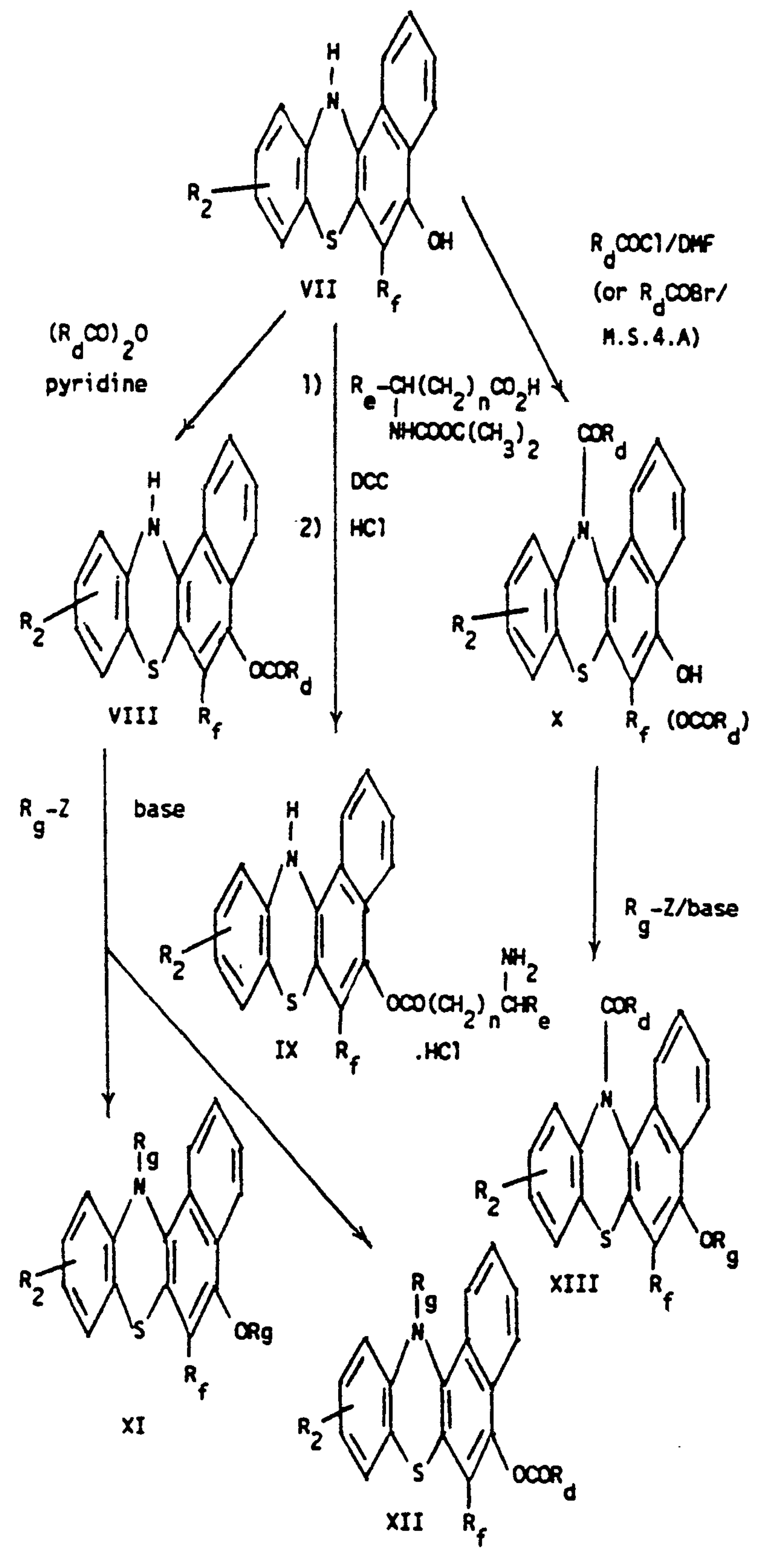

SCHEME II

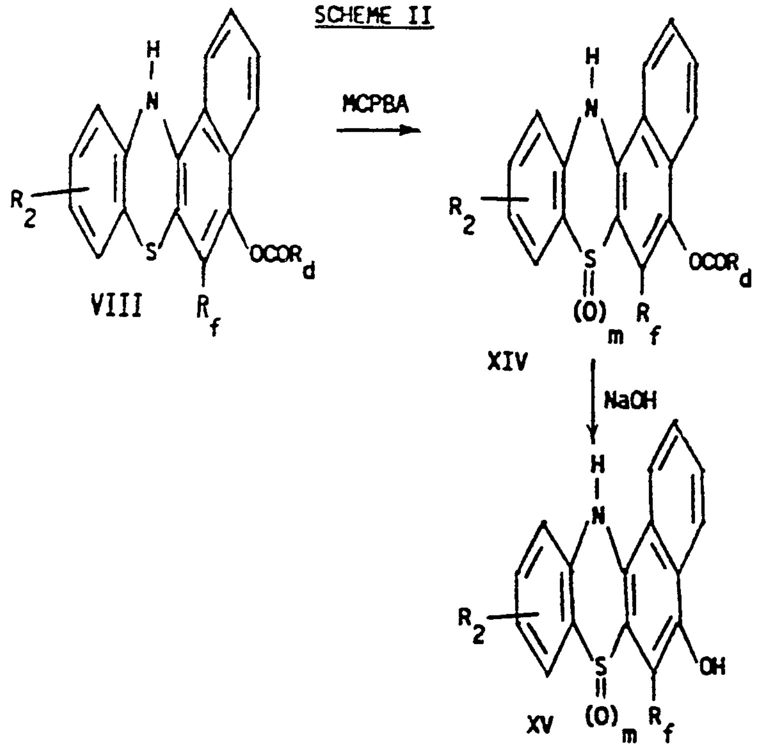


SCHEME III

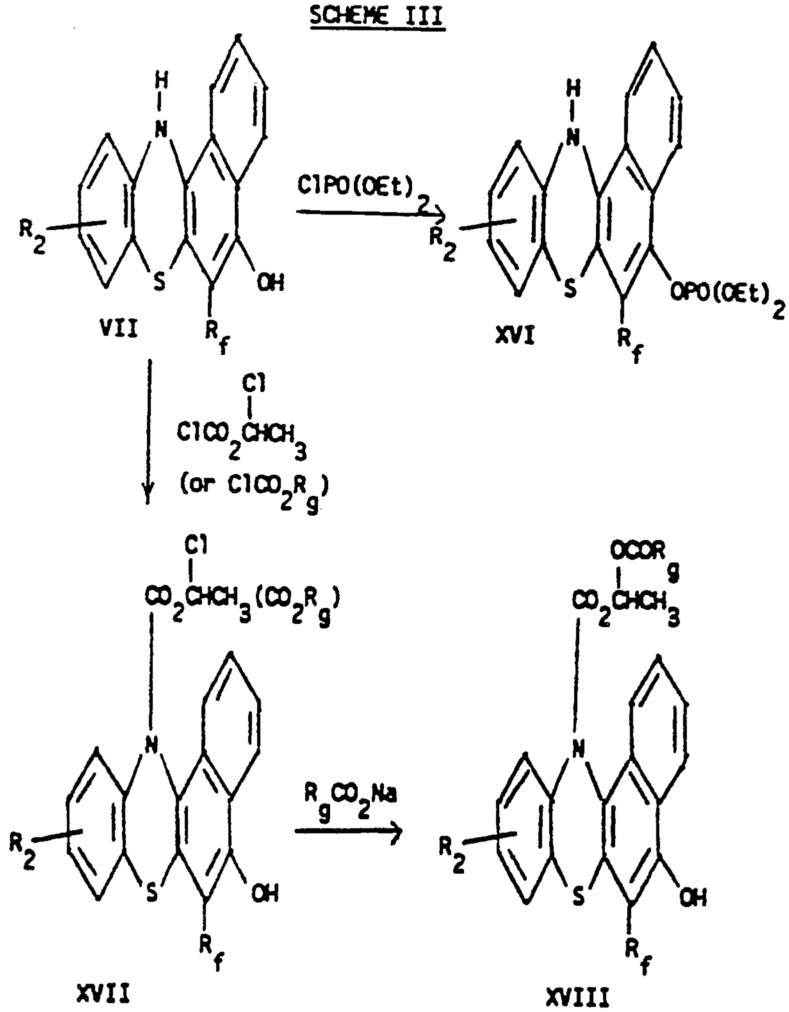

where:

$R_d$ is $C_1$ to $C_4$ alkyl or phenyl;

$R_e$ is a residue such that the reagent

$$R_e—\underset{\displaystyle NHCOOC(CH_3)_2}{\underset{|}{C}H}(CH_2)_nCO_2H;$$

is a derivative of an essential amino acid:

m is 1 or 2;

n is 0 to 3;

$R_f$ is hydrogen, $C_1$ to $C_4$ lower alkyl, $C_1$ to $C_5$ acyloxy or hydroxy;

$R_g$ is $C_1$ to $C_5$ lower alkyl or benzyl;

Z is Cl, Br, I, tosylate or mesylate.

M.S.4.A is 4 Ångstrøm molecular sieves; and MCPBA is m-chloroperbenzoic acid.

## SCHEME IV

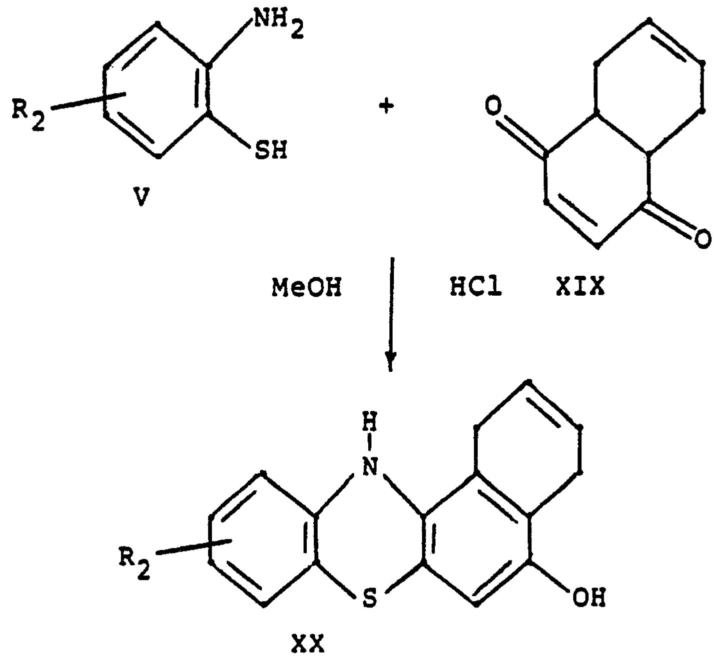


V

XIX

XX

SCHEME IV (Continued)

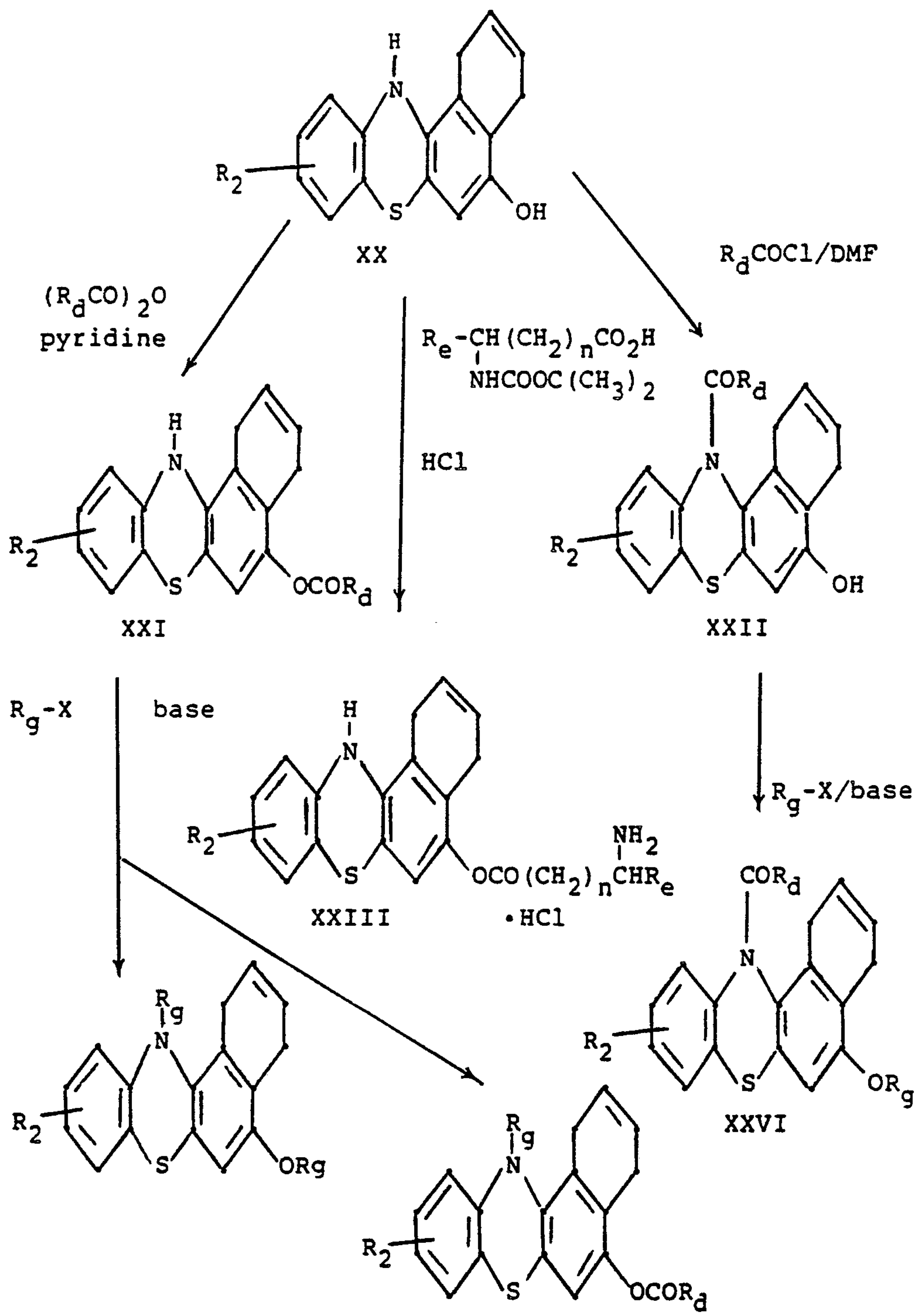


XX, XXI, XXII, XXIII, XXIV, XXV, XXVI

Reaction of a naphthoquinone IV, optimally two equivalents, with a 2-aminobenzenethiol V, in a solvent such as acetic acid, acetic acid-water, or a lower alkanol at from −20° to +60°C for 0.25 to 6 hours yields the benzo[a]phenothiazin-5-one VI. Preferably, the solvent is methanol or ethanol, at 0° to 25°C for 0.5 to 2 hours. Reduction of VI to VII is carried out with a reducing agent such as sodium hydrosulfite in a suitable solvent system by stirring at from 10° to 50°C (preferably at room temperature) for 1 to 12 hours (preferably 1 to 4 hours). The solvent system may be a homogeneous one such as dimethylformamide-water or dichlormethane-water (Scheme I).

The O-acyl compounds VIII are prepared by reacting compound VII with the desired acid anhydride in pyridine at a temperature of from −25° to +75°C (preferably 0° to 50°C) for from 1 hour to 24 hours (preferably 4 to 15 hours) (Scheme I).

The O-aminoacyl compounds IX are prepared from VIII by reacting the latter with a N-blocked amino acid and dicyclohexylcarbodiimide (DCC), followed by removal of the blocking group by treatment with HCl (Scheme I).

The N-acyl compound X can be prepared from VII by reacting the latter with an acyl halide, such as acetyl chloride, in a solvent such as dimethyl formamide at from 0° to 50°C (preferably room temperature) for from 0.5 to 4 hours depending on the rate of reaction of the particular components (Scheme I).

To obtain the N,O-dialkyl compounds XI compound VIII is reacted with an alkyl halide or a methyl sulfonate (preferably an alkyl iodide) in the presence of a strong base such as sodium hydride or potassium t-butoxide in a solvent such as tetrahydrofuran or dimethylformamide at 0° to 60°C (preferably room temperature) for from 1 to 24 hours (preferably 1 to 10 hours).

In this same reaction, some of the N-alkyl-O-acyl compound XII is also obtained, and is separated from XI by chromatography. The N-acyl-O-alkyl compounds XIII are prepared from compound X by a procedure similar to that used to prepare XI and XII (Scheme I).

The sulfoxide derivative XIV (m=1) are prepared by treating VIII with peracid such as peracetic acid or meta-chloroperbenzoic acid (MCPBA), in a solvent such as methylene chloride or methylene chloride-methanol for 0.5 to 4 hours at 0° to 30°C. The sulfones XIV (m=2) are obtained by reacting VIII with a peracid in methylene chloride-methanol, or preferably 1,2 dichloro-ethane-ethanol, at reflux temperature of the mixture for 12 to 24 hours, depending upon the rate of reaction. Hydrolysis of XIV to XV is carried out by reaction with a base (for example LiOH, NaOH or KOH) in a mixed solvent such as methanol-water or ethanol-water, at from 0° to 60°C (preferably room temperature) for from 5 minutes to 180 minutes (preferably 10 minutes to 90 minutes) (Scheme II).

To prepare a carbamate derivative such as XVII, compound VII is reacted with the appropriate chloroformate reagent in a suitable solvent such as tetrahydrofuran, dioxane or preferably acetonitrile and the mixture heated to reflux for 4 to 24 hours. Reaction of the appropriate chloroalkylcarbamate XVII with a metal salt of a carboxylic acid then yields the acyloxyalkoxycarbonyl compound XVIII. Preferred salts are those of silver, mercury (II) or sodium, using the corresponding free acid as a solvent, and heating the reaction mixture at 0° to 100°C for from 10 minutes to 2 hours (Scheme III).

The 1,4-dihydro series of compounds (XX) is prepared by condensing a 2-amino benzenethiol V with diketone XIX under conditions similar to those used for the reaction between IV and V. Preparation of compounds XXI to XXVI is carried out by methodology very similar to that described in Scheme I to obtain compounds VIII to XIII (Scheme IV).

Some of the benzo[a]-phenothiazin-5-one derivatives used as starting materials are described in European Patent Application nr. 84300239.5, published on August 8, 1984 under publication number EP—A—0115394 (Attorney Docket No. 16876Y).

It will be evident to one skilled in the art that $R_2$ must be chosen so as to be compatible with the reaction outlined in Schemes I to IV.

The following examples are provided to illustrate the invention. All temperatures are reported in degrees Celsius (°C) and are uncorrected.

### Example 1

Preparation of 5-acetoxy-12H-benzo[a]phenothiazine

Acetic anhydride (2 ml) was added to a solution of 5-hydroxy-12H-benzo[a]phenothiazine (1) (1.4 gm) in pyridine (10 ml) and stirred for 15 minutes. The reaction mixture was concentrated under vacuum and ice-water was added to the residue. The resulting precipitate was filtered, air-dried and washed with ethyl acetate to afford the title compound.

| | | | | | |
|---|---|---|---|---|---|
| m.p. 184—5° Anal. $C_{18}H_{13}NO_2S$ Calcd.: | C, 70.34; | H, 4.26; | N, 4.56; | S, 10.43. |
| Found: | C, 70.28; | H, 4.32; | N, 4.53; | S, 10.51. |

Ref. (1): C.A. 70:69169z. S. African spec. nr. 6801,996.

### Example 2

Preparation of 5-methoxy-12-methyl-12H-benzo[a]phenothiazine and 5-acetoxy-12-methyl-12H-benzo[a]phenothiazine

Potassium tert. butoxide (1.3 gm) was added to a solution of 5-acetoxy-12H-benzo[a]phenothiazine (from Example 1) (2.0 gm) and methyl iodide (4 ml) in DMF (20 ml). The reaction mixture was cooled with an ice-bath and stirred for 15 minutes. Diethyl ether (100 ml) was added to the reaction mixture followed by brine (100 ml). The ether layer was decanted, washed with brine, dried and evaporated to dryness. The resulting oily residue was chromatographed on silica gel (10% EtOAc/hexane) to afford 5-methoxy-12-methyl-12H-benzo[a]phenothiazine.

m.p. 123—4°, Anal. $C_{18}H_{15}NOS$ Calcd.: C, 76.69; H, 5.15; N, 4.77; S, 10.93.
Found: C, 73.64; H, 5.30; N, 4.71; S, 11.00.

followed by 5-acetoxy-12-methyl-12H-benzo[a]phenothiazine.

m.p. 109—110°, Anal. $C_{19}H_{15}NO_2S$ Calcd.: C, 71.01; H, 4.70; N, 4.36; S, 9.98.
Found: C, 71.25; H, 4.90; N, 4.26; S, 10.12.

Example 3

Preparation of 12-acetyl-5-hydroxy-12H-benzo[a]-phenothiazine

Acetyl chloride (2 ml) was added to a solution of 5-hydroxy-12H-benzo[a]phenothiazine (1) (2.65 gm) in DMF (10 ml) and stirred for 30 minutes. Diethyl ether (100 ml) was added to the reaction mixture followed by ice-water (50 ml). The aqueous layer was decanted and the organic layer containing a solid was evaporated to dryness. The resulting residue was treated with acetone and filtered to afford the title compound.

m.p. 230°. Anal. $C_{18}H_{13}NO_2S$. Calcd.: C, 70.34; H, 4.26; N, 4.56; S, 10.43.
Found: C, 70.43; H, 4.38; N, 4.55; S, 10.62.
Ref. (1): C.A. 70:69169z. S. African Spec. nr. 6801,966.

Example 4

Preparation of 12-Acetyl-5-methoxy-12H-benzo[a]phenothiazine

Potassium tert-butoxide (800 mg) was added to a solution of 12-acetyl-5-hydroxy-12H-benzo[a]phenothiazine (950 mg) (from Example 3) and methyl iodide (1.5 ml) in DMF (10 ml) and stirred for 15 minutes. Water (80 ml) was added to the reaction mixture and the precipitate was filtered to afford the title compound.

m.p. 208—9° Anal. $C_{19}H_{15}NO_2S$ Calcd.: C, 71.01; H, 4.70; N, 4.36; S, 9.98.
Found: C, 70.97; H, 4.84; N, 4.30; S, 10.07.

Example 5

Preparation of 5-aminoacetoxy-12H-benzo[a]phenothiazine-hydrochloride salt

Dicyclohexylcarbodiimide (21 g) was added to a solution of 5-hydroxy-12H-benzo[a]phenothiazine (4.2 g) and N-tert-butyloxycarbonyl glycine (8.4 g) in THF (150 ml) followed by the addition of DMAP (0.2 g). The reaction mixture was stirred for 15 minutes, filtered and the filtrate evaporated to dryness. The residue was dissolved in EtOAc, washed with a solution of $NaHCO_3$, dried and evaporated to dryness. The resulting oily residue was chromatographed on silica gel (5% $EtOAc/CH_2Cl_2$) to afford 5-tert-butyloxy-carbonylamino acetoxy-12H-benzo[a]phenothiazine. m.p. 175° as an intermediate.

The above intermediate (1 g) was dissolved in $CH_2Cl_2$ (35 ml) cooled to 0° and HCl (gas) was bubbled into the solution for 15 minutes. The precipitate was filtered to afford the title compound.

m.p. 189° Anal. $C_{18}H_{13}N_2O_2S.HCl$ Calcd.: C, 60.25; H, 4.21; N, 7.80; S, 8.93; Cl, 9.88.
Found: C, 60.00; H, 4.30; N, 7.90; S, 8.78; Cl, 10.16.

Example 6

Preparation of 5-Hydroxy-1,4-dihydro-12H-benzo[a]phenothiazine

A solution of 2-aminothiophenol (385 mg) in methanol (3 ml) was added to a solution of 4a,5,8,8a-tetrahydro-1,4-naphthoquinone (Ref.: Ber. *62,* 2361 (1929)) (500 mg) in methanol (10 ml) and stirred for 1 hour. Then conc. HCl (2 ml) was added and the mixture was stirred for another 2 hours. Ethyl acetate was added to the reaction mixture followed by a solution of $NaHCO_3$. The organic layer was decanted, dried and evaporated to dryness. The resulting oily residue was chromatographed on silica gel ($CH_2Cl_2$) to afford the title compound.

m.p. 166° Anal. $C_{16}H_{13}NOS$ Calcd.: C, 71.88; H, 4.90; N, 5.23; S, 11.99.
Found: C, 71.74; H, 4.88; N, 5.25; S, 12.17.

Example 7

Preparation of 5-Hydroxy-6-methyl-12H-benzo[a]phenothiazine and 5-acetoxy-6-methyl-12H-benzo[a]phenothiazine

A solution of sodium hydrosulfite (40 g) in water (0.5 l) was added to a suspension of 6-methyl-5H-benzo[a]phenothiazine-5-one (10.4 g) in ethyl acetate (1 l) and stirred for 2 hours. The organic layer was decanted, dried and evaporated to dryness. The resulting residue was triturated with ether and filtered to afford 5-hydroxy-6-methyl-12H-benzo[a]phenothiazine as an air sensitive solid.

The above compound (3 gm) was dissolved in a mixture of pyridine (20 ml) and acetic anhydride (10 ml) and stirred for 15 minutes. The reaction mixture was concentrated under vacuum and the residue triturated with diethyl ether and filtered. A sample was chromatographed on silica gel ($CH_2Cl_2$) to afford the title compound.

m.p. 170°, Anal. $C_{19}H_{15}NO_2S$ Calcd.: C, 71.00; H, 4.70; N, 4.35; S, 9.97.
Found: C, 70.95; H, 4.80; N, 4.24; S, 10.14.

Example 8

Preparation of 5-Benzoyloxy-12H-benzo[a]phenothiazine

Following the procedure of Example 1 but substituting benzoic anhydride for acetic anhydride, the title compound was obtained.

| | | | | |
|---|---|---|---|---|
| m.p. 171°C. Anal. $C_{13}H_{15}NO_2S$; Calcd.: | C, 74.78; | H, 4.09; | N, 3.79; | S, 8.68. |
| Found: | C, 74.93; | H, 4.22; | N, 3.96; | S, 8.57. |

Example 9

Preparation of 5-Trimethylacetoxy-12H-benzo[a]phenothiazine

Following the procedure of Example 1 but substituting trimethyl acetic anhydride for acetic anhydride, the title compound was obtained.

| | | | | |
|---|---|---|---|---|
| m.p. 142°C. Anal. $C_{21}H_{19}NO_2S$; Calcd.: | C, 72.18; | H, 5.48; | N, 4.01; | S, 9.17. |
| Found: | C, 72.15; | H, 5.46; | N, 4.21; | S, 9.34. |

Example 10

Preparation of 5-Methoxymethoxy-12H-benzo[a]phenothiazine

Sodium hydride (75 mg) was added to a solution of 5-hydroxy-12H-benzo[a]phenothiazine (1) (500 mg) and chloromethyl ether (154 mg) in THF (10 ml) and the reaction mixture was stirred for 15 minutes. Diethyl ether (60 ml) was added to the reaction mixture followed by water (60 ml). The ether layer was decanted, dried and evaporated to dryness. The resulting residue was chromatographed on silica gel ($CH_2Cl_2$/hexane (7:3)) to afford the title compound, m.p. 103°C.

Anal. $C_{18}H_{15}NO_2S$;

Calcd: C, 69.87; H, 4.89; N, 4.53; S, 10.36.

Found: C, 69.75; H, 5.01; N, 4.41; S, 10.53.

Example 11

Synthesis of 5-Acetoxy-12-acetyl-12H-benzo[a]phenothiazine

To a mixture of 5-acetoxy-12H-benzo[a]phenothiazine (3.0 g) and powdered 4 Angstrom molecular sieves (7.5 g) in 1,2-dichloroethane (75 ml) there were added slowly acetyl bromide (1.05 ml). The mixture was stirred at room temperature for 1 hour, then filtered. The filtrate was evaporated down to an oil which was triturated with a mixture of ether and hexane to afford solid title product (3 g), m.p. 141—142°C.

Example 12

Synthesis of 5-Methoxy-12H-benzo[a]phenothiazine

To a solution of 5-hydroxy-12H-benzo[a]phenothiazine (2.65 g) and methyl iodide (2.5 ml) in DMF (dimethylformamide) (10 ml) there was added powdered potassium carbonate (2.0 g). The mixture was stirred at room temperature and after 15 minutes, another addition of pottasium carbonate (2.0 g) and methyl iodide (2.0 ml) was done. The mixture was stirred for a further 20 minutes, then it was diluted with ethyl acetate (100 ml and washed with water (60 ml) twice, dried and evaporated down. The residue was triturated with ether to afford solid crude product which was filtered (1.7 g). This crude product was chromatographed on a column of silica gel, eluting with dichloromethane to afford pure title compound (1.33 g), m.p. 168—170°C.

Example 13

Synthesis of Diethyl 12H-benzo[a]phenothiazin-5-yl phosphate

To a solution of 5-hydroxy-12H-benzo[a]phenothiazine (1.5 g) and diethyl chlorophosphate (2 ml) in DMF (20 ml) there was added a powdered mixture of potassium carbonate (2 g) and potassium iodide (2 g). The reaction mixture was stirred at room temperature. After 30 minutes, a further addition of potassium carbonate and potassium iodide (2 g each) was made and stirring was continued. Two hours later more carbonate (2 g) and diethyl chlorophosphate (2 ml) were added, and then three hours later a final addition of carbonate (2 g) was made. The mixture was stirred for 3 days then diluted with ethyl acetate (60 ml) and the insolubles filtered. The filtrate was washed with water, dried and evaporated down. This residue was crystallized from acetone, then chromatographed on a column of silica gel, eluting first with 1:1, then 3:1 mixtures of ethyl acetate and hexane to afford pure title compound (337 mg), m.p. 162—163°C.

Example 14

Synthesis of 5-Hydroxy-12-(1-acetoxyethoxycarbonyl)-12H-benzo[a]phenothiazine

*Step 1:* Preparation of α-Chloroethyl chloroformate

To a mixture of ethyl chloroformate (108.5 g) and sulfuryl chloride (138 g), benzoyl peroxide (1 g) was added and it was refluxed for 20 hours. The reaction mixture was distilled and the liquid boiling above 110° was collected. This was then fractionated using a 30 cm column packed with glass helices to give 32 g of pure alpha-chloroethyl chloroformate, b.p. 118—119°.

*Step 2:* Preparation of 5-Hydroxy-12-(α-chloroethoxycarbonyl)-12H-benzo[a]phenothiazine

A mixture of 5-hydroxy-12H-benzo[a]phenothiazine (5 g) and α-chloroethyl chloroformate (10 g) in tetrahydrofuran (20 ml) was refluxed for 9 hours, then the volatile components were evaporated away and the residue chromatographed on a column of silica gel, eluting with a 1:9 ethyl acetate-hexane mixture to afford the title compound (4 g) as an oil.

*Step 3:* Preparation of 5-Hydroxy-12-(1-acetoxyethoxycarbonyl)-12H-benzo[a]phenothiazine

The product from Step 2 (4 g) and sodium acetate (6 g) were refluxed together in glacial acetic acid (60 ml) for 1 hour. The mixture was cooled, diluted with ethyl acetate (300 ml) and washed with water (3 × 100 ml), then with aqueous sodium bicarbonate and again with water, dried and evaporated down. The residue was chromatographed on a column of silica gel eluting with a 1:9 mixture of ethyl acetate-hexane to afford, after trituration in ether and filtration, the pure title compound (1.2 g), m.p. 195—196°C.

Example 15

Synthesis of 5-Acetoxy-9-methoxy-12H-benzo[a]phenothiazine

By following the procedures described in preparative Example 7, but substituting 9-methoxy-5H-benzo[a]phenothiazine-5-one for the 6-methyl analog as starting material, the title compound was obtained, m.p. 156—158°C.

Example 16

Synthesis of 5-Acetoxy-9-methyl-12H-benzo[a]phenothiazine

By following the procedures described in preparative Example 7, but substituting 9-methyl-5H-benzo[a]phenothiazine-5-one for the 6-methyl analog as starting material, the title compound was prepared, m.p. 193—195°C.

Example 17

Synthesis of 5-Acetoxy-9-fluoro-12H-benzo[a]phenothiazine

By following the procedures described in preparative Example 7, but substituting 9-fluoro-5H-benzo[a]phenothiazine-5-one for the 6-methyl analog as starting material, the title compound was obtained, m.p. 196—198°C (dec.).

Example 18

Synthesis of 5-Acetoxy-12H-benzo[a]phenoxazine

Applying the procedures described in preparative Example 7, but substituting 5H-benzo[a]phenoxazine-5-one for 6-methyl-5H-benzo[a]phenothiazine-5-one, the title compound was synthesized, m.p. 152—155°C.

Example 19

Synthesis of 5,6-Diacetoxy-12H-benzo[a]phenothiazine

By following the procedures described in preparative Example 7, but substituting 6-acetoxy-5H-benzo[a]phenothiazine-5-one for the 6-methyl analog as starting material there was obtained the title compound, m.p. 206—207°C.

Example 20

Synthesis of 12-Acetyl-5,6-diacetoxy-12H-benzo[a]phenothiazine

By following the procedure described in preparative Example 11, but substituting 5,6-diacetoxy-12H-benzo[a]phenothiazine for the 5-acetoxy analog as starting material, the title compound was obtained, m.p. 213—214°C.

Example 21

Synthesis of 12-Acetyl-5,6-dihydroxy-12H-benzo[a]phenothiazine

To a suspension of 12-acetyl-5,6-diacetoxy-12H-benzo[a]phenothiazine (450 mg) in acetone (10 ml) there was added 2N aqueous sodium hydroxide solution (10 ml) and the resulting mixture was stirred at room temperature for 45 minutes. It was then made slightly acidic by the addition of 10% aqueous acetic acid solution and the insolubles filtered and washed with water. The solid was swished in ethyl acetate to afford pure title compound (100 mg), m.p. 258—260°C.

Example 22

Synthesis of 5-Acetoxy-12H-benzo[a]phenothiazine-7-oxide

To a suspension of 5-acetoxy-12H-benzo[a]phenothiazine (10 g) in dichloromethane (125 cc) there was rapidly added a solution of 85% m-chloroperoxybenzoic acid (6.61 g) in methanol (125 ml). At first the solids dissolved, then after a few minutes a new solid separated out of solution. After 1 and ½ hours the mixture was filtered and the solid washed with dichloromethane. The title compound was thus obtained pure (8.76 g), m.p. 179—181°C.

Example 23

Synthesis of 5-Acetoxy-12H-benzo[a]phenothiazine-7,7-dioxide

To a suspension of 5-acetoxy-12H-benzo[a]phenothiazine (10 g) in dichloromethane (125 ml) there was rapidly added a solution of 85% m-chloroperoxybenzoic acid (18 g) in methanol (125 ml). A solution resulted which rapidly began to deposit the sulfoxide. The mixture was heated to a gentle reflux and slowly the solid redissolved, then the sulfone began to crystallize out of solution. After 2 and ½ hours the mixture was allowed to cool down and it was filtered to afford nearly pure title product. A 1 gram sample was crystallized from THF (tetrahydrofuran) to afford pure product (504 mg), m.p. 284—287°C.

Example 24

Synthesis of 5-Hydroxy-12H-benzo[a]phenothiazine-7,7-dioxide

To a suspension of 5-acetoxy-12H-benzo[a]phenothiazine-7,7-dioxide (6.6 g) in methanol (200 ml) there was added 2N aqueous sodium hydroxide solution (132 ml) and the mixture was stirred in the absence of air at room temperature for 7 minutes; at that point an amber solution had resulted. There was rapidly added 10% aqueous acetic acid solution (200 ml) causing precipitation of the title compound which was collected by filtration (5.68 g). A sample (500 mg) was crystallized from THF affording purified product (250 mg), m.p. 334°C (dec).

Example 25

Synthesis of 5,6-Dihydroxy-12H-benzo[a]phenothiazine-7,7-dioxide.

To a suspension of 6-hydroxy-5H-benzo[a]phenothiazine-5-one-7,7-dioxide (430 mg) in water (10 cc) and ethyl acetate (10 ml) there was added sodium dithionite (1 g). The suspension was stirred vigorously at room temperature for 2 hours, then the insolubles were filtered and washed with water and ethyl acetate. There was obtained 346 mg of the title compound, m.p. >330°C.

Example 26

Synthesis of 5-Acetoxy-12-(1-acetoxyethoxycarbonyl)-12H-benzo[a]phenothiazine

By following the procedure of Example 1, but substituting the product of Example 14 for 5-hydroxy-12H-benzo[a]phenothiazine, the title compound was obtained, m.p. 76—78°C.

Example 27

Synthesis of 5-Hydroxy-12-(1-acetoxyethoxycarbonyl)-12H-benzo[a]phenoxazine

By following the procedure of Example 14, Steps 2 and 3, but substituting 5-hydroxy-12H-benzo[a]phenoxazine for 5-hydroxy-12H-benzo[a]phenothiazine, the title compound is obtained.

Certain of the compounds of the present invention contain one or more centers of asymmetry. This invention is meant to include both the racemic and the resolved forms of such compounds.

**Claims**

1. A pharmaceutical composition capable of inhibiting leukotriene biosynthesis or action in mammals, especially humans, and containing a pharmaceutically acceptable carrier and a compound having the Formula I or a compound that is a pharmaceutically acceptable salt of a compound of Formula I:

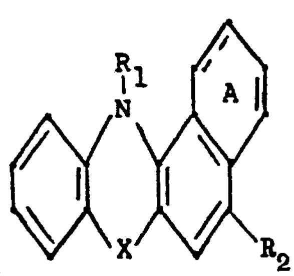

I

in which

X is O, S, SO or $SO_2$

$R_1$ is H; $C_{1-6}$ alkyl; $C_{1-6}$ acyl; or $COOR_7$, where $R_7$ is ($C_{1-6}$ acyloxy)-($C_{1-6}$ alkyl);

$R_2$ is (a) hydrogen, (b) —$OPO(OR_6)_2$ where $R_6$ is H, phenyl or $C_{1-6}$ alkyl or (c) $OR_a$ where $R_a$ is H; $C_{1-5}$ alkyl; $C_{1-5}$ acyl; ($C_{1-6}$ alkoxy)-($C_{1-6}$ alkyl); or $C_{1-4}$ aminoacyl;

and the broken lines in ring A indicate that it may be an aromatic or 1,4-dihydroaromatic ring.

2. A composition as claimed in Claim 1, in which, in the formula, X is S.

3. A composition as claimed in Claim 1, in which, in the formula, X is O.

4. A composition as claimed in Claim 2, in which, in the formula, X is S and at least one of $R_1$ and $R_a$ is other than hydrogen.

5. A pharmaceutical composition capable of inhibiting leukotriene biosynthesis or action in mammals, especially humans, and containing a pharmaceutically acceptable carrier and a compound having the Formula I or a compound that is a pharmaceutically acceptable salt of a compound of Formula I:

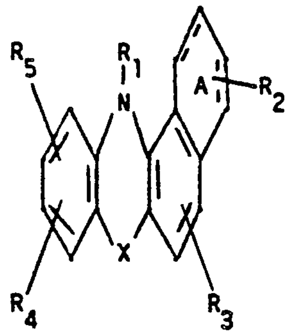

I

in which the compound has one of the following substituent patterns:

| X | Ring A | $R_1$ | $R_2$ | $R_3$ | $R_4$ | $R_5$ |
|---|---|---|---|---|---|---|
| S | aromatic | H | 5-OH | H | H | H |
| S | aromatic | H | 5-$OCOCH_3$ | 6-Cl | H | H |
| S | aromatic | H | H | H | H | H |
| S | aromatic | H | 5-OH | 6-Cl | H | H |
| S | aromatic | H | 5-OH | 6-Cl | 9-Cl | H |
| S | aromatic | H | 5-OH | 6-Cl | 9-$OCH_3$ | H |
| S | aromatic | H | 5-$OCOCH_3$ | 6-Cl | 9-$CH_3$ | H |
| O | aromatic | H | 5-OH | H | H | H |
| O | aromatic | H | 5-$OCOCH_3$ | H | H | H |
| SO | aromatic | H | 5-OH | H | H | H |
| $SO_2$ | aromatic | H | 5-OH | H | H | H |
| SO | aromatic | H | H | H | H | H |
| $SO_2$ | aromatic | H | H | H | H | H |
| SO | aromatic | $COCH_3$ | H | H | H | H |
| S | aromatic | H | 1-$CO_2H$ | H | H | H |
| S | aromatic | $CH_3$ | 2-Et | H | H | H |
| S | aromatic | $COCH_3$ | 2-$COCH_3$ | H | H | H |
| S | aromatic | H | 2-$COCH_3$ | H | H | H |
| S | aromatic | H | H | H | H | 10-phenyl |
| S | aromatic | H | H | H | 8-$CH_3$ | 11-$CH_3$ |
| S | aromatic | H | H | H | H | 10-$COCH_3$ |
| S | aromatic | H | 5-OH | 6-$NHCOCH_3$ | 9-F | H |
| S | aromatic | H | 5-OH | 6-NH-phenyl | 9-Cl | H |
| SO | aromatic | H | 5-OAc | 6-Cl | H | 10-$CF_3$ |
| SO | aromatic | H | 5-$OCH_2Ph$ | 6-Cl | H | 10-$CF_3$ |

| X | Ring A | $R_1$ | $R_2$ | $R_3$ | $R_4$ | $R_5$ |
|---|---|---|---|---|---|---|
| S | aromatic | H | 5-OH | 6-Cl | 9-$CH_3$ | 11-$CH_3$ |
| S | aromatic | H | 5-$OCH_3$ | 6-Cl | 9-$OCH_3$ | H |
| $SO_2$ | 1,4-dihydro | H | 5-OH | H | H | H |
| $SO_2$ | aromatic | $COCH_3$ | 4-$CH_3$ | H | 9-$SCH_3$ | H |
| O | aromatic | H | H | H | 9-$SO_2CF_3$ | H |
| O | aromatic | H | H | H | 11-$CH_3$ | H |
| S | aromatic | $COCH_3$ | 5-OCOMe | H | H | H |
| S | aromatic | $CH_2OAc$ | 5-OH | H | H | H |
| S | aromatic | $CH_2OAc$ | 5-OCOMe | H | H | H |
| S | aromatic | Me | 5-OH | H | H | H |
| S | aromatic | H | 5-$OCOCH_3$ | H | H | H |
| S | aromatic | $CH_3$ | 5-$OCH_3$ | H | H | H |
| S | aromatic | $CH_3$ | 5-$OCOCH_3$ | H | H | H |
| S | aromatic | $COCH_3$ | 5-OH | H | H | H |
| S | aromatic | $COCH_3$ | 5-$OCH_3$ | H | H | H |
| S | aromatic | H | 5-$OCOCH_2NH_2$ | H | H | H |
| S | 1,4-dihydro | H | 5-OH | H | H | H |
| S | aromatic | H | 5-$OCOCH(CH_3)_2$ | H | H | H |
| S | aromatic | H | 5-$OCOC(CH_3)_3$ | H | H | H |
| S | aromatic | H | 5-$OCOC_6H_5$ | H | H | H |
| S | aromatic | H | 5-OH | 6-$CH_3$ | H | H |
| S | aromatic | H | 5-$OCOCH_3$ | 6-$CH_3$ | H | H |
| S | aromatic | H | 5-OH | 1-OH | 6-$CH_3$ | H |
| S | aromatic | H | 5-$OCOCH_3$ | 1-OH | 6-$CH_3$ | H |
| S | aromatic | H | 5-OH | 1-$OCH_3$ | 6-$CH_3$ | H |
| S | aromatic | H | 5-$OCOCH_3$ | 1-$OCH_3$ | 6-$CH_3$ | H |
| S | aromatic | H | 5-$OCOC(CH_3)_3$ | 1-$OCH_3$ | 6-$CH_3$ | H |
| S | 1,4-dihydro | H | 5-$OCOCH_3$ | H | H | H |
| $SO_2$ | aromatic | H | 5-$OCOCH_3$ | H | H | H |
| SO | aromatic | H | 5-$OCOCH_3$ | H | H | H |
| $SO_2$ | aromatic | H | 5-$OCOCH_3$ | H | H | H |
| S | aromatic | H | 5-$OCH_2OCH_3$ | H | H | H |

| X | Ring A | $R_1$ | $R_2$ | $R_3$ | $R_4$ | $R_5$ |
|---|---|---|---|---|---|---|
| S | aromatic | H | 5-$OCH_3$ | H | H | H |
| S | aromatic | H | 5-$OCOC_6H_4$—p—OMe | H | H | H |
| S | aromatic | H | 5-$OCOC_6H_4$-p-Cl | H | H | H |
| S | aromatic | H | 5-$OCOC_6H_4$—p—$NH_2$ | H | H | H |
| S | aromatic | H | 5-$OCOC_6H_4$—p—$CO_2H$ | H | H | H |
| S | aromatic | H | 5-$OCOC_6H_4$—O—$CF_3$ | H | H | H |
| S | aromatic | H | 5-$OCOC_6H_4$—m—CN | H | H | H |
| S | aromatic | H | 5-$OCOC(CH_2)_2CO_2H$ | H | H | H |
| S | aromatic | H | 5-$OCO(CH_2)_4CO_2H$ | H | H | H |
| S | aromatic | H | 5-$OCOCH(NH_2)CH_3$ | H | H | H |
| S | aromatic | H | 5-$OCOCH(NH_2)CH_2$—<br>$C_6H_5$ | H | H | H |
| S | aromatic | $CO_2CHCH_3$<br>OAc | 5-OHA | H | H | H |
| S | aromatic | $CO_2CHCH_3$<br>OAc | 5-OAc | H | H | H |
| S | aromatic | $CO_2CHCH_3$<br>OAc | 5-$OCO_2CHCH_3$<br>OAc | H | H | H |
| S | aromatic | H | 5-$OCO_2CHCH_3$<br>OAc | H | H | H |
| S | aromatic | H | 5-$OPO(OEt)_2$ | H | H | H |
| S | aromatic | H | 5-OH | 9-OMe | H | H |
| S | aromatic | H | 5-OAc | 9-OMe | H | H |
| S | aromatic | H | 5-OH | 9-Me | H | H |
| S | aromatic | H | 5-OAc | 9-Me | H | H |
| S | aromatic | H | 5-OH | 9-F | H | H |
| S | aromatic | H | 5-OAc | 9-F | H | H |
| S | aromatic | H | 5-OH | 6-OAc | H | H |
| S | aromatic | H | 5-OAc | 6-OAc | H | H |
| S | aromatic | Ac | 5-OH | 6-OH | H | H |
| S | aromatic | Ac | 5-OAc | 6-OAc | H | H |
| $SO_2$ | aromatic | H | 5-OH | 6-OH | H | H |

| X | Ring A | $R_1$ | $R_2$ | $R_3$ | $R_4$ | $R_5$ |
|---|---|---|---|---|---|---|
| O | aromatic | OAc<br>$CO_2CHCH_3$ | 5-OH | H | H | H |
| $SO_2$ | aromatic | OAc<br>$CO_2CHCH_3$ | 5-OH | H | H | H |

6. A composition as claimed in Claim 1, in which, in the formula, X is SO.

7. A composition as claimed in Claim 1, in which, in the formula, X is $SO_2$.

8. A composition as claimed in Claim 1, for use in (a) inhibiting mammalian leukotriene biosynthesis or action; (b) treating cardiovascular disorders; (c) treating inflammation; (d) treating allergies; (e) treating pain; or (f) treating psoriasis in a mammal in need of relief.

9. A compound having the formula:

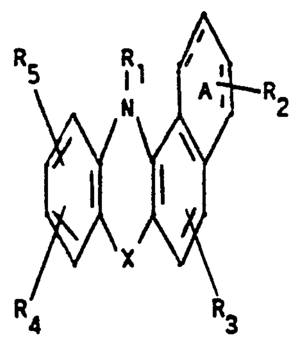

and one of the following substituent patterns:

| X | Ring A | $R_1$ | $R_2$ | $R_3$ | $R_4$ | $R_5$ |
|---|---|---|---|---|---|---|
| S | aromatic | H | 5-$OCOCH_3$ | H | H | H |
| S | aromatic | $CH_3$ | 5-$OCH_3$ | H | H | H |
| S | aromatic | $CH_3$ | 5-$OCOCH_3$ | H | H | H |
| S | aromatic | $COCH_3$ | 5-OH | H | H | H |
| S | aromatic | $COCH_3$ | 5-$OCH_3$ | H | H | H |
| S | aromatic | H | 5-$OCOCH_2NH_2$ | H | H | H |
| S | 1,4-dihydro | H | 5-OH | H | H | H |
| S | aromatic | H | 5-$OCOCH(CH_3)_2$ | H | H | H |
| S | aromatic | H | 5-$OCOC(CH_3)_3$ | H | H | H |
| S | aromatic | H | 5-$OCOC_6H_5$ | H | H | H |
| S | aromatic | H | 5-OH | 6-$CH_3$ | H | H |
| S | aromatic | H | 5-$OCOCH_3$ | 6-$CH_3$ | H | H |
| S | aromatic | H | 5-OH | 1-OH | 6-$CH_3$ | H |
| S | aromatic | H | 5-$OCOCH_3$ | 1-OH | 6-$CH_3$ | H |
| S | aromatic | H | 5-OH | 1-$OCH_3$ | 6-$CH_3$ | H |
| S | aromatic | H | 5-$OCOCH_3$ | 1-$OCH_3$ | 6-$CH_3$ | H |
| S | aromatic | H | 5-$OCOC(CH_3)_3$ | 1-$OCH_3$ | 6-$CH_3$ | H |

| X | Ring A | $R_1$ | $R_2$ | $R_3$ | $R_4$ | $R_5$ |
|---|---|---|---|---|---|---|
| S | 1,4-dihydro | H | 5-$OCOCH_3$ | H | H | H |
| S | aromatic | H | 5-$OCOCH_3$ | H | H | H |
| $SO_2$ | aromatic | H | 5-$OCOCH_3$ | H | H | H |
| SO | aromatic | H | 5-OH | H | H | H |
| $SO_2$ | aromatic | H | 5-OH | H | H | H |
| S | aromatic | H | 5-$OCH_2OCH_3$ | H | H | H |
| S | aromatic | H | 5-$OCH_3$ | H | H | H |
| S | aromatic | $COCH_3$ | 5-$OCOCH_3$ | H | H | H |
| S | aromatic | $CH_3$ | 5-OH | H | H | H |
| S | aromatic | $CO_2CH(OAc)CH_3$ | 5-OH | H | H | H |
| S | aromatic | $CO_2CH(OAc)CH_3$ | 5-OAc | H | H | H |
| S | aromatic | $CO_2CH(OAc)CH_3$ | 5-$OCO_2CH(OAc)CH_3$ | H | H | H |
| S | aromatic | H | 5-$OCO_2CH(OAc)CH_3$ | H | H | H |
| S | aromatic | H | 5-$OPO(OEt)_2$ | H | H | H |
| S | aromatic | H | 5-OH | 9-OMe | H | H |
| S | aromatic | H | 5-OAc | 9-OMe | H | H |
| S | aromatic | H | 5-OH | 9-Me | H | H |
| S | aromatic | H | 5-OAc | 9-Me | H | H |
| S | aromatic | H | 5-OH | 9-F | H | H |
| S | aromatic | H | 5-OAc | 9-F | H | H |
| S | aromatic | H | 5-OH | 6-OAc | H | H |
| S | aromatic | H | 5-OAc | 6-OAc | H | H |
| S | aromatic | Ac | 5-OH | 6-OH | H | H |
| S | aromatic | Ac | 5-OAc | 6-OAc | H | H |
| $SO_2$ | aromatic | H | 5-OH | 6-OH | H | H |
| O | aromatic | H | 5-OH | H | H | H |
| O | aromatic | H | 5-OAc | H | H | H |

| X | Ring A | $R_1$ | $R_2$ | $R_3$ | $R_4$ | $R_5$ |
|---|---|---|---|---|---|---|
| O | aromatic | $CO_2CH(OAc)CH_3$ | 5-OH | H | H | H |
| $SO_2$ | aromatic | $CO_2CH(OAc)CH_3$ | 5-OH | H | H | H |

10. 5-Acetoxy-12H-benzo[a]phenothiazine.
11. 5-Hydroxy-12-(1-acetoxyethoxycarbonyl)-12H-benzo[a]phenothiazine.
12. 5-Acetoxy-12H-benzo[a]phenothiazine-7,7-dioxide.
13. 5-Hydroxy-12H-benzo[a]phenothiazine-7,7-dioxide.
14. 5-Acetoxy-12-(1-acetoxyethoxycarbonyl)-12H-benzo[a]phenothiazine.
15. 5-Acetoxy-12H-benzo[a]phenoxazine.
16. 5-Hydroxy-12-(1-acetoxyethoxycarbonyl)12H-benzo[a]phenoxazine.
17. A composition as claimed in any one of Claims 1 to 7, containing an effective amount of additional second active ingredient that is a non-steroidal anti-inflammatory drug; a peripheral analgesic agent; a cyclooxygenase inhibitor; a leukotriene antagonist; a leukotriene inhibitor; an $H_2$-receptor antagonist; an antihistaminic agent; a prostaglandin antagonist or a thromboxane antagonist.
18. A composition as claimed in Claim 17, in which the second active ingredient is an non-steroidal anti-inflammatory drug.
19. A composition as claimed in Claim 18, in which the non-steroidal anti-inflammatory drug in indomethancin.
20. A composition as claimed in Claims 17 to 19, in which the weight ratio of the compound defined in Claim 1 to the second active ingredient ranges from 1000:1 to 1:1000.
21. A composition as claimed in Claim 20, in which the said ratio ranges from 200:1 to 1:200.

**Patentansprüche**

1. Pharmazeutische Zusammensetzung, die die Leukotrienbiosynthese oder -wirkung in Säugetieren, insbesondere beim Menschen, inhibieren kann und einen pharmazeutisch annehmbaren Träer und eine Verbindung der Formel I oder eine Verbindung, die ein pharmazeutisch annehmbares Salz einer Verbindung der Formel I ist, enthält

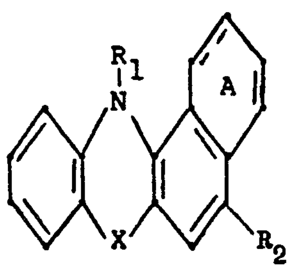

I

worin

X O, S, SO oder $So_2$ ist;

$R_1$ H; $C_{1-6}$-Alkyl; $_{1-6}$-Acyl; oder $COOR_7$ ist, worin

$R_7$ ($C_{1-6}$-Acyloxy)-($C_{1-6}$-alkyl) ist;

$R_2$ (a) Wasserstoff, (b) —$OPO(OR_6)_2$, worin

$R_6$ H, Phenyl oder $C_{1-6}$-Alkyl ist, oder (c) $OR_a$ ist, worin

$R_a$ H; $C_{1-5}$-Alkyl; $C_{1-5}$-Acyl; ($C_{1-6}$-Alkoxy)-($C_{1-6}$-alkyl); oder $C_{1-4}$-Aminoacyl ist; und die durchbrochenen Linien in Ring A anzeigen, daß es sich um einen aromatischen oder 1,4-dihydroaromatischen Ring handelt.

2. Zusammensetzung, wie in Anspruch 1 beansprucht, worin in der Formel X S ist.
3. Zusammensetzung, wie in Anspruch 1 beansprucht, worin in der Formel X O ist.
4. Zusammensetzung, wie in Anspruch 2 beansprucht, worin in der Formel X S is und wenigstens eines aus $R_1$ und $R_a$ von Wasserstoff verschieden ist.
5. Pharmazeutische Zusammensetzung, die die Leukotrienbiosynthese oder -wirkung in Säugetieren, insbesondere beim Menschen, inhibieren kann und einen pharmazeutisch annehmbaren Träger und eine Verbindung der Formel I oder eine Verbindung, die ein pharmazeutisch annehmbares Salz einer Verbindung der Formel I ist, enthält

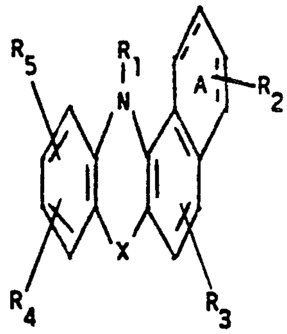

I

worin die Verbindung eines der folgenden Substitutionsmuster aufweist:

| X | Ring A | $R_1$ | $R_2$ | $R_3$ | $R_4$ | $R_5$ |
|---|---|---|---|---|---|---|
| S | aromatisch | H | 5—OH | H | H | H |
| S | aromatisch | H | 5—$OCOCH_3$ | 6—Cl | H | H |
| S | aromatisch | H | H | H | H | H |
| S | aromatisch | H | 5—OH | 6—Cl | H | H |
| S | aromatisch | H | 5—OH | 6—Cl | 9—Cl | H |
| S | aromatisch | H | 5—OH | 6—Cl | 9—$OCH_3$ | H |
| S | aromatisch | H | 5—$OCOCH_3$ | 6—Cl | 9—$CH_3$ | H |
| O | aromatisch | H | 5—OH | H | H | H |
| O | aromatisch | H | 5—$OCOCH_3$ | H | H | H |
| SO | aromatisch | H | 5—OH | H | H | H |
| $SO_2$ | aromatisch | H | 5—OH | H | H | H |
| SO | aromatisch | H | H | H | H | H |
| $SO_2$ | aromatisch | H | H | H | H | H |
| SO | aromatisch | $COCH_3$ | H | H | H | H |
| S | aromatisch | H | 1—$CO_2H$ | H | H | H |
| S | aromatisch | $CH_3$ | 2—Et | H | H | H |
| S | aromatisch | $COCH_3$ | 2—$COCH_3$ | H | H | H |
| S | aromatisch | H | 2—$COCH_3$ | H | H | H |
| S | aromatisch | H | H | H | H | 10-phenyl |
| S | aromatisch | H | H | H | 6—$CH_3$ | 11—$CH_3$ |
| S | aromatisch | H | H | H | H | 10—$COCH_3$ |
| S | aromatisch | H | 5—OH | 6—$NHCOCH_3$ | 9—F | H |
| SO | aromatisch | H | 5—OH | 6—NH-phenyl | 9—Cl | H |
| SO | aromatisch | H | 5—OAc | 6—Cl | H | 10—$CF_3$ |
| SO | aromatisch | H | 5—$OCH_2Ph$ | 5—Cl | H | 10—$CF_3$ |

| X | Ring A | $R_1$ | $R_2$ | $R_3$ | $R_4$ | $R_5$ |
|---|---|---|---|---|---|---|
| S | aromatisch | H | 5—OH | 6—Cl | 9—$CH_3$ | 11—$CH_3$ |
| S | aromatisch | H | 5—$OCH_3$ | 6—Cl | 9—$OCH_3$ | H |
| $SO_2$ | 1,4-Dihydro | H | 5—OH | H | H | H |
| $SO_2$ | aromatisch | $COCH_3$ | 4—$CH_3$ | H | 9—$SCH_3$ | H |
| O | aromatisch | H | H | H | 9—$SO_2CF_3$ | H |
| O | aromatisch | H | H | H | 11—$CH_3$ | H |
| S | aromatisch | $COCH_3$ | 5—OCOMe | H | H | H |
| S | aromatisch | $CH_2OAc$ | 5—OH | H | H | H |
| S | aromatisch | $CH_2OAc$ | 5—OCOMe | H | H | H |
| S | aromatisch | Me | 5—OH | H | H | H |
| S | aromatisch | H | 5—$OCOCH_3$ | H | H | H |
| S | aromatisch | $CH_3$ | 5—$OCH_3$ | H | H | H |
| S | aromatisch | $CH_3$ | 5—$OCOCH_3$ | H | H | H |
| S | aromatisch | $COCH_3$ | 5—OH | H | H | H |
| S | aromatisch | $COCH_3$ | 5—$OCH_3$ | H | H | H |
| S | aromatisch | H | 5—$OCOCH_2NH_2$ | H | H | H |
| S | 1,4-Dihydro | H | 5—OH | H | H | H |
| S | aromatisch | H | 5—$OCOCH(CH_3)_2$ | H | H | H |
| S | aromatisch | H | 5—$OCOC(CH_3)_3$ | H | H | H |
| S | aromatisch | H | 5—$OCOC_6H_5$ | H | H | H |
| S | aromatisch | H | 5—OH | 6—$CH_3$ | H | H |
| S | aromatisch | H | 5—$OCOCH_3$ | 6—$CH_3$ | H | H |
| S | aromatisch | H | 5—OH | 1—OH | 6—$CH_3$ | H |
| S | aromatisch | H | 5—$OCOCH_3$ | 1—OH | 6—$CH_3$ | H |
| S | aromatisch | H | 5—OH | 1—$OCH_3$ | 6—$CH_3$ | H |
| S | aromatisch | H | 5—$OCOCH_3$ | 1—$OCH_3$ | 6—$CH_3$ | H |
| S | aromatisch | H | 5—$OCOC(CH_3)_3$ | 1—$OCH_3$ | 6—$CH_3$ | H |
| S | 1,4-Dihydro | H | 5—$OCOCH_3$ | H | H | H |
| $SO_2$ | aromatisch | H | 5—$OCOCH_3$ | H | H | H |
| SO | aromatisch | H | 5—$OCOCH_3$ | H | H | H |
| $SO_2$ | aromatisch | H | 5—$OCOCH_3$ | H | H | H |

| X | Ring A | $R_1$ | $R_2$ | $R_3$ | $R_4$ | $R_5$ |
|---|---|---|---|---|---|---|
| S | aromatisch | H | 5-$OCH_2OCH_3$ | H | H | H |
| S | aromatisch | H | 5-$OCH_3$ | H | H | H |
| S | aromatisch | H | 5-$OCOC_6H_4$—p—OMe | H | H | H |
| S | aromatisch | H | 5-$OCOC_6H_4$—p—Cl | H | H | H |
| S | aromatisch | H | 5-$OCOC_6H_4$—p—$NH_2$ | H | H | H |
| S | aromatisch | H | 5-$OCOC_6H_4$—p—$CO_2H$ | H | H | H |
| S | aromatisch | H | 5-$OCOC_6H_4$—O—$CF_3$ | H | H | H |
| S | aromatisch | H | 5-$OCOC_6H_4$—m—CH | H | H | H |
| S | aromatisch | H | 5-$OCO(CH_2)_2CO_2H$ | H | H | H |
| S | aromatisch | H | 5-$OCO(CH_2)_4CO_2H$ | H | H | H |
| S | aromatisch | H | 5-$OCOCH(NH_2)CH_3$ | H | H | H |
| S | aromatisch | H | 5-$OCOCH(NH_2)CH_2$—$C_6H_5$ | H | H | H |
| S | aromatisch | $CO_2CHCH_3$ (CH—OAc) | 5-OH | H | H | H |
| S | aromatisch | $CO_2CHCH_3$ (CH—OAc) | 5-OAc | H | H | H |
| S | aromatisch | $CO_2CHCH_3$ (CH—OAc) | 5-$OCO_2CHCH_3$ (CH—OAc) | H | H | H |
| S | aromatisch | H | 5-$OCO_2CHCH_3$ (CH—OAc) | H | H | H |
| S | aromatisch | H | 5-$OPO(OEt)_2$ | H | H | H |
| S | aromatisch | H | 5-OH | 9-OMe | H | H |
| S | aromatisch | H | 5-OAc | 9-OMe | H | H |
| S | aromatisch | H | 5-OH | 9-Me | H | H |
| S | aromatisch | H | 5-OAc | 9-Me | H | H |
| S | aromatisch | H | 5-OH | 9-F | H | H |
| S | aromatisch | H | 5-OAc | 9-F | H | H |
| S | aromatisch | H | 5-OH | 6-OAc | H | H |
| S | aromatisch | H | 5-OAc | 6-OAc | H | H |
| S | aromatisch | Ac | 5-OH | 6-OH | H | H |
| S | aromatisch | Ac | 5-OAc | 6-OAc | H | H |
| $SO_2$ | aromatisch | H | 5-OH | 6-OH | H | H |

| X | Ring A | $R_1$ | $R_2$ | $R_3$ | $R_4$ | $R_5$ |
|---|---|---|---|---|---|---|
| O | aromatisch | OAc<br>$CO_2CHCH_3$ | 5-OH | H | H | H |
| $SO_2$ | aromatisch | OAc<br>$CO_2CHCH_3$ | 5-OH | H | H | H |

6. Zusammensetzung, wie in Anspruch 1 beansprucht, worin in der Formel X SO ist.

7. Verbindung, wie in Anspruch 1 beansprucht, worin in der Formel X $SO_2$ ist.

8. Zusammensetzung, wie in Anspruch 1 beansprucht, zur Verwendung bei (a) der Inhibierung der Leukotrienbiosynthese oder -wirkung in Säuretieren; (c) der Behandlung von kardiovaskularen Zuständen; (c) der Behandlung von Entzündungen; (d) der Behandlung von Allergien; (e) der Behandlung von Schmerz; oder (f) der Behandlung von Psoriasis bei einem Säugetier, das der Erleichterung bedarf.

9. Verbindung der Formel

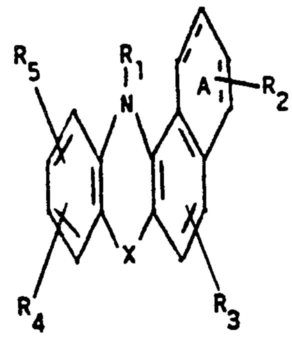

mit einem der folgenden Substitutionsmuster:

| X | Ring A | $R_1$ | $R_2$ | $R_3$ | $R_4$ | $R_5$ |
|---|---|---|---|---|---|---|
| S | aromatisch | H | 5-$OCOCH_3$ | H | H | H |
| S | aromatisch | $CH_3$ | 5-$OCH_3$ | H | H | H |
| S | aromatisch | $CH_3$ | 5-$OCOCH_3$ | H | H | H |
| S | aromatisch | $COCH_3$ | 5-OH | H | H | H |
| S | aromatisch | $COCH_3$ | 5-$OCH_3$ | H | H | H |
| S | aromatisch | H | 5-$OCOCH_2NH_2$ | H | H | H |
| S | 1,4-Dihydro | H | 5-OH | H | H | H |
| S | aromatisch | H | 5-$OCOCH(CH_3)_2$ | H | H | H |
| S | aromatisch | H | 5-$OCOC(CH_3)_3$ | H | H | H |
| S | aromatisch | H | 5-$OCOC_6H_5$ | H | H | H |
| S | aromatisch | H | 5-OH | 6-$CH_3$ | H | H |
| S | aromatisch | H | 5-$OCOCH_3$ | 6-$CH_3$ | H | H |
| S | aromatisch | H | 5-OH | 1-CH | 6-$CH_3$ | H |
| S | aromatisch | H | 5-$OCOCH_3$ | 1-CH | 6-$CH_3$ | H |
| S | aromatisch | H | 5-OH | 1-$OCH_3$ | 6-$CH_3$ | H |
| S | aromatisch | H | 5-$OCOCH_3$ | 1-$OCH_3$ | 6-$CH_3$ | H |

| X | Ring A | $R_1$ | $R_2$ | $R_3$ | $R_4$ | $R_5$ |
|---|---|---|---|---|---|---|
| S | aromatisch | H | 5-$OCOC(CH_3)_3$ | 1-$OCH_3$ | 6-$CH_3$ | H |
| S | 1,4-Dihydro | H | 5-$OCOCH_3$ | H | H | H |
| S | aromatisch | H | 5-$OCOCH_3$ | H | H | H |
| $SO_2$ | aromatisch | H | 5-$OCOCH_3$ | H | H | H |
| SO | aromatisch | H | 5-OH | H | H | H |
| $SO_2$ | aromatisch | H | 5-OH | H | H | H |
| S | aromatisch | H | 5-$OCH_2OCH_3$ | H | H | H |
| S | aromatisch | H | 5-$OCH_3$ | H | H | H |
| S | aromatisch | $COCH_3$ | 5-$OCOCH_3$ | H | H | H |
| S | aromatisch | $CH_3$ | 5-OH | H | H | H |
| S | aromatisch | $CO_2CH(OAc)CH_3$ | 5-OH | H | H | H |
| S | aromatisch | $CO_2CH(OAc)CH_3$ | 5-OAc | H | H | H |
| S | aromatisch | $CO_2CH(OAc)CH_3$ | 5-$OCO_2CH(OAc)CH_3$ | H | H | H |
| S | aromatisch | H | 5-$OCO_2CH(OAc)CH_3$ | H | H | H |
| S | aromatisch | H | 5-$OPO(OEt)_2$ | H | H | H |
| S | aromatisch | H | 5-OH | 9-OMe | H | H |
| S | aromatisch | H | 5-OAc | 9-OMe | H | H |
| S | aromatisch | H | 5-OH | 9-Me | H | H |
| S | aromatisch | H | 5-OAc | 9-Me | H | H |
| S | aromatisch | H | 5-OH | 9-F | H | H |
| S | aromatisch | H | 5-OAc | 9-F | H | H |
| S | aromatisch | H | 5-OH | 6-OAc | H | H |
| S | aromatisch | H | 5-OAc | 6-OAc | H | H |
| S | aromatisch | Ac | 5-OH | 6-OH | H | H |
| S | aromatisch | Ac | 5-OAc | 6-OAc | H | H |
| SO | aromatisch | H | 5-OH | 6-OH | H | H |
| O | aromatisch | H | 5-OH | H | H | H |
| O | aromatisch | H | 5-OAc | H | H | H |

| X | Ring A | $R_1$ | $R_2$ | $R_3$ | $R_4$ | $R_5$ |
|---|---|---|---|---|---|---|
| O | aromatisch | OAc<br>\|<br>$CO_2CHCH_3$ | 5-OH | H | H | H |
| $SO_2$ | aromatisch | OAc<br>\|<br>$CO_2CHCH_3$ | 5-OH | H | H | H |

10. 5-Acetoxy-12H-benzo[a]phenothiazin.
11. 5-Hydroxy-12-(1-acetoxyethoxycarbonyl)-12H-benzo[a]phenothiazin.
12. 5-Acetoxy-12H-benzo[a]phenothiazin-7,7-dioxid.
13. 5-Hydroxy-12H-benzo[a]phenothiazin-7,7-dioxid.
14. 5-Acetoxy-12-(1-acetoxyethoxycarbonyl)-12H-benzo[a]phenothiazin.
15. 5-Acetoxy-12H-benzo[a]phenoxazin.
16. 5-Hydroxy-12-[1-acetoxyethoxycarbonyl)-12H-benzo[a]phenoxazin.
17. Zusammensetzung, wie in einem der Ansprüche 1 bis 7 beansprucht, welche eine wirksame Menge eines zusätzlichen zweiten aktiven Bestandteils enthält, der ein nichtsteroides, entzündungshemmendes Mittel, eine peripheres analgetisches Mittel, ein Cyclooxygenaseinhibitor, ein Leukotrienantagonist; ein Leukotrieninhibitor; ein $H_2$-Rezeptor-Antagonist; ein Antihistaminikum; ein Prostaglandinantagonist oder ein Thromboxanantagonist ist.
18. Zusammensetzung, wie in Anspruch 17 beansprucht, worin der zweite aktive Bestandteil ein nichtsteroides entzündungshemmendes Mittel ist.
19. Zusammensetzung, wie in Anspruch 18 beansprucht, worin das nichtsteroide entzündungshemmende Mittel Indomethazin ist.
20. Zusammensetzung, wie in Anspruch 17 bis 19 beansprucht, worin das Gewichtsverhältnis der in Anspruch 1 definierten Verbindung zum zweiten aktiven Bestandteil im Bereich von 1000:1 bis 1:1000 liegt.
21. Zusammensetzung, wie in Anspruch 20 beansprucht, worin das Verhältnis im Bereich von 200:1 bis 1:200 liegt.

**Revendications**

1. Composition pharmaceutique capable d'inhiber la biosynthèse ou l'action des leucotriènes chez les mammifères, en particulier chez l'homme, et contenant un véhicule pharmaceutiquement acceptable et un composé répondant à la formule I ou un composé qui est un sel pharmaceutiquement acceptable d'un composé répondant à la formule I:

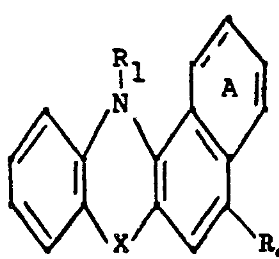

I

dans laquelle

X est O, S, SO ou $SO_2$;

$R_1$ est H; un groupe alkyle en $C_{1-6}$; un groupe acyle en $C_{1-6}$; ou $COOR_7$, où $R_7$ est un groupe (acyloxy en $C_{1-6}$)-(alkyle en $C_{1-6}$);

$R_2$ est (a) un atome d'hydrogène, (b) $-OPO(OR_6)_2$ où

$R_6$ est H, un groupe phényle ou alkyle en $C_{1-6}$ ou (c) $OR_a$ où

$R_a$ est H; un groupe alkyle en $C_{1-5}$; un groupe acyle en $C_{1-5}$; un groupe (alcoxy en $C_{1-6}$)-(alkyle en $C_{1-6}$); ou un groupe aminoacyle en $C_{1-4}$;

et les lignes en traits interrompus dans le noyau A indique qu'il peut être un noyau aromatique ou aromatique 1,4-dihydrogéné.

2. Composition selon la revendication 1, dans laquelle, dans la formule, X est S.
3. Composition selon la revendication 1, dans laquelle, dans la formule, X est O.
4. Composition selon la revendication 2, dans laquelle, dans la formule, X est S et au moins un parmi $R_1$ et $R_a$ est différent de l'hydrogène.
5. Composition pharmaceutique capable d'inhiber la biosynthèse ou l'action des leucotriènes chez les mammifères, en particulier chez l'homme, et contenant un véhicule pharmaceutiquement acceptable et un composé répondant à la formule I ou un composé qui est un sel pharmaceutiquement acceptable d'un composé répondant à la formule I:

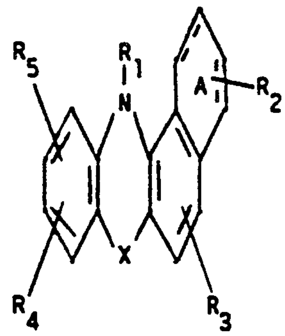

I

dans laquelle le composé possède l'un des modèles suivants de substituants:

| X | Noyau A | $R_1$ | $R_2$ | $R_3$ | $R_4$ | $R_5$ |
|---|---|---|---|---|---|---|
| S | aromatique | H | 5-OH | H | H | H |
| S | aromatique | H | 5-$OCOCH_3$ | 6-Cl | H | H |
| S | aromatique | H | H | H | H | H |
| S | aromatique | H | 5-OH | 6-Cl | H | H |
| S | aromatique | H | 5-OH | 6-Cl | 9-Cl | H |
| S | aromatique | H | 5-OH | 6-Cl | 9-$OCH_3$ | H |
| S | aromatique | H | 5-$OCOCH_3$ | 6-Cl | 9-$CH_3$ | H |
| O | aromatique | H | 5-OH | H | H | H |
| O | aromatique | H | 5-$OCOCH_3$ | H | H | H |
| SO | aromatique | H | 5-OH | H | H | H |
| $SO_2$ | aromatique | H | 5-OH | H | H | H |
| SO | aromatique | H | H | H | H | H |
| $SO_2$ | aromatique | H | H | H | H | H |
| SO | aromatique | $COCH_3$ | H | H | H | H |
| S | aromatique | H | 1-$CO_2H$ | H | H | H |
| S | aromatique | $CH_3$ | 2-Et | H | H | H |
| S | aromatique | $COCH_3$ | 2-$COCH_3$ | H | H | H |
| S | aromatique | H | 2-$COCH_3$ | H | H | H |
| S | aromatique | H | H | H | H | 10-phényl |
| S | aromatique | H | H | H | 8-$CH_3$ | 11-$CH_3$ |
| S | aromatique | H | H | H | H | 10-$COCH_3$ |
| S | aromatique | H | 5-OH | 6-$NHCOCH_3$ | 9-F | H |
| S | aromatique | H | 5-OH | 6-NH-phényl | 9-Cl | H |
| SO | aromatique | H | 5-OAc | 6-Cl | H | 10-$CF_3$ |

| X | Noyau A | $R_1$ | $R_2$ | $R_3$ | $R_4$ | $R_5$ |
|---|---|---|---|---|---|---|
| SO | aromatique | H | 5-$OCH_2Ph$ | 6-Cl | H | 10-$CF_3$ |
| S | aromatique | H | 5-OH | 6-Cl | 9-$CH_3$ | 11-$CH_3$ |
| S | aromatique | H | 5-$OCH_3$ | 6-Cl | 9-$OCH_3$ | H |
| $SO_2$ | 1,4-dihydro | H | 5-OH | H | H | H |
| $SO_2$ | aromatique | $COCH_3$ | 4-$CH_3$ | H | 9-$SCH_3$ | H |
| O | aromatique | H | H | H | 9-$SO_2CF_3$ | H |
| O | aromatique | H | H | H | 11-$CH_3$ | H |
| S | aromatique | $COCH_3$ | 5-OCOMe | H | H | H |
| S | aromatique | $CH_2OAc$ | 5-OH | H | H | H |
| S | aromatique | $CH_2OAc$ | 5-OCOMe | H | H | H |
| S | aromatique | Me | 5-OH | H | H | H |
| S | aromatique | H | 5-$OCOCH_3$ | H | H | H |
| S | aromatique | $CH_3$ | 5-$OCH_3$ | H | H | H |
| S | aromatique | $CH_3$ | 5-$OCOCH_3$ | H | H | H |
| S | aromatique | $COCH_3$ | 5-OH | H | H | H |
| S | aromatique | $COCH_3$ | 5-$OCH_3$ | H | H | H |
| S | aromatique | H | 5-$OCOCH_2NH_2$ | H | H | H |
| S | 1,4-dihydro | H | 5-OH | H | H | H |
| S | aromatique | H | 5-$OCOCH(CH_3)_2$ | H | H | H |
| S | aromatique | H | 5-$OCOC(CH_3)_3$ | H | H | H |
| S | aromatique | H | 5-$OCOC_6H_5$ | H | H | H |
| S | aromatique | H | 5-CH | 6-$CH_3$ | H | H |
| S | aromatique | H | 5-$OCOCH_3$ | 6-$CH_3$ | H | H |
| S | aromatique | H | 5-OH | 1-OH | 6-$CH_3$ | H |
| S | aromatique | H | 5-$OCOCH_3$ | 1-OH | 6-$CH_3$ | H |
| S | aromatique | H | 5-OH | 1-$OCH_3$ | 6-$CH_3$ | H |
| S | aromatique | H | 5-$OCOCH_3$ | 1-$OCH_3$ | 6-$CH_3$ | H |
| S | aromatique | H | 5-$OCOC(CH_3)_3$ | 1-$OCH_3$ | 6-$CH_3$ | H |
| S | 1,4-dihydro | H | 5-$OCOCH_3$ | H | H | H |
| $SO_2$ | 1,4-dihydro | H | 5-$OCOCH_3$ | H | H | H |
| SO | aromatique | H | 5-$OCOCH_3$ | H | H | H |
| $SO_2$ | aromatique | H | 5-$OCOCH_3$ | H | H | H |

| X | Noyau A | $R_1$ | $R_2$ | $R_3$ | $R_4$ | $R_5$ |
|---|---|---|---|---|---|---|
| S | aromatique | H | 5-OCH2O$CH_3$ | H | H | H |
| S | aromatique | H | 5-$OCH_3$ | H | H | H |
| S | aromatique | H | 5-$OCOC_6H_4$—p—OMe | H | H | H |
| S | aromatique | H | 5-$OCOC_6H_4$—p—Cl | H | H | H |
| S | aromatique | H | 5-$OCOC_6H_4$—p—$NH_2$ | H | H | H |
| S | aromatique | H | 5-$OCOC_6H_4$—p—$CO_2H$ | H | H | H |
| S | aromatique | H | 5-$OCOC_6H_4$—O—$CF_3$ | H | H | H |
| S | aromatique | H | 5-$OCOC_6H_4$—m—CN | H | H | H |
| S | aromatique | H | 5-$OCO(CH_2)_2$—$CO_2H$ | H | H | H |
| S | aromatique | H | 5-$OCO(CH_2)_4$—$CO_2H$ | H | H | H |
| S | aromatique | H | 5-$OCOCH(NH_2)CH_3$ | H | H | H |
| S | aromatique | H | 5-$OCOCH(NH_2)$—$CH_2C_6H_5$ | H | H | H |
| S | aromatique | $CO_2CHCH_3$ (\| OAc) | 5-OH | H | H | H |
| S | aromatique | $CO_2CHCH_3$ (\| OAc) | 5-OAc | H | H | H |
| S | aromatique | $CO_2CHCH_3$ (\| OAc) | 5-$OCO_2CHCH_3$ (\| OAc) | H | H | H |
| S | aromatique | H | 5-$OCO_2CHCH_3$ (\| OAc) | H | H | H |
| S | aromatique | H | 5-$OPO(OEt)_2$ | H | H | H |
| S | aromatique | H | 5-OH | 9-OMe | H | H |
| S | aromatique | H | 5-OAc | 9-OMe | H | H |
| S | aromatique | H | 5-OH | 9-Me | H | H |
| S | aromatique | H | 5-OAc | 9-Me | H | H |
| S | aromatique | H | 5-OH | 9-F | H | H |
| S | aromatique | H | 5-OAc | 9-F | H | H |
| S | aromatique | H | 5-OH | 6-OAc | H | H |
| S | aromatique | H | 5-OAc | 6-OAc | H | H |
| S | aromatique | Ac | 5-OH | 6-OH | H | H |
| S | aromatique | Ac | 5-OAc | 6-OAc | H | H |
| $SO_2$ | aromatique | H | 5-OH | 5-OH | H | H |

| X | Noyau A | $R_1$ | $R_2$ | $R_3$ | $R_4$ | $R_5$ |
|---|---|---|---|---|---|---|
| O | aromatique | OAc<br>\|<br>$CO_2CHCH_3$ | 5-OH | H | H | H |
| $SO_2$ | aromatique | OAc<br>\|<br>$CO_2CHCH_3$ | 5-OH | H | H | H |

6. Composition selon la revendication 1, dans laquelle, dans la formule, X est SO.

7. Composition selon la revendication 1, dans laquelle, dans la formule, X est $SO_2$.

8. Composition selon la revendication 1, à utiliser pour (a) l'inhibition de la biosynthèse ou de l'action des leucotriènes chez les mammifères; (b) le traitement des troubles cardivasculaires; (c) le traitement de l'inflammation; (d) le traitement des allergies; (e) le traitement de la douleur; ou (f) le traitement du psoriasis chez un mammifère ayant besoin d'être soulagé.

9. Composé répondant à la formule

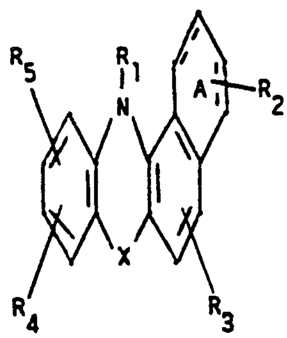

et ayant l'un des modèles suivants de substituants:

| X | Noyau A | $R_1$ | $R_2$ | $R_3$ | $R_4$ | $R_5$ |
|---|---|---|---|---|---|---|
| S | aromatique | H | 5-$OCOCH_3$ | H | H | H |
| S | aromatique | $CH_3$ | 5-$OCH_3$ | H | H | H |
| S | aromatique | $CH_3$ | 5-$OCOCH_3$ | H | H | H |
| S | aromatique | $COCH_3$ | 5-OH | H | H | H |
| S | aromatique | $COCH_3$ | 5-$OCH_3$ | H | H | H |
| S | aromatique | H | 5-$OCOCH_2NH_2$ | H | H | H |
| S | 1,4-dihydro | H | 5-OH | H | H | H |
| S | aromatique | H | 5-$OCOCH(CH_3)_2$ | H | H | H |
| S | aromatique | H | 5-$OCOC(CH_3)_3$ | H | H | H |
| S | aromatique | H | 5-$OCOC_6H_5$ | H | H | H |
| S | aromatique | H | 5-OH | 6-$CH_3$ | H | H |
| S | aromatique | H | 5-$OCOCH_3$ | 6-$CH_3$ | H | H |
| S | aromatique | H | 5-OH | 1-OH | 6-$CH_3$ | H |
| S | aromatique | H | 5-$OCOCH_3$ | 1-OH | 6-$CH_3$ | H |
| S | aromatique | H | 5-OH | 1-$OCH_3$ | 6-$CH_3$ | H |
| S | aromatique | H | 5-$OCOCH_3$ | 1-$OCH_3$ | 6-$CH_3$ | H |

| X | Noyau A | $R_1$ | $R_2$ | $R_3$ | $R_4$ | $R_5$ |
|---|---|---|---|---|---|---|
| S | aromatique | H | 5-$OCOC(CH_3)_3$ | 1-$OCH_3$ | 6-$CH_3$ | H |
| S | 1,4-dihydro | H | 5-$OCOCH_3$ | H | H | H |
| SO | aromatique | H | 5-$OCOCH_3$ | H | H | H |
| $SO_2$ | aromatique | H | 5-$OCOCH_3$ | H | H | H |
| SO | aromatique | H | 5-OH | H | H | H |
| $SO_2$ | aromatique | H | 5-OH | H | H | H |
| S | aromatique | H | 5-$OCH_2OCH_3$ | H | H | H |
| S | aromatique | H | 5-$OCH_3$ | H | H | H |
| S | aromatique | $COCH_3$ | 5-$OCOCH_3$ | H | H | H |
| S | aromatique | $CH_3$ | 5-OH | H | H | H |
| S | aromatique | $CO_2CH(OAc)CH_3$ | 5-OH | H | H | H |
| S | aromatique | $CO_2CH(OAc)CH_3$ | 5-OAc | H | H | H |
| S | aromatique | $CO_2CH(OAc)CH_3$ | 5-$OCO_2CH(OAc)CH_3$ | H | H | H |
| S | aromatique | H | 5-$OCO_2CH(OAc)CH_3$ | H | H | H |
| S | aromatique | H | 5-$OPO(OEt)_2$ | H | H | H |
| S | aromatique | H | 5-OH | 9-OMe | H | H |
| S | aromatique | H | 5-OAc | 9-OMe | H | H |
| S | aromatique | H | 5-OH | 9-Me | H | H |
| S | aromatique | H | 5-OAc | 9-Me | H | H |
| S | aromatique | H | 5-OH | 9-F | H | H |
| S | aromatique | H | 5-OAc | 9-F | H | H |
| S | aromatique | H | 5-OH | 6-OAc | H | H |
| S | aromatique | H | 5-OAc | 6-OAc | H | H |
| S | aromatique | Ac | 5-OH | 6-OH | H | H |
| S | aromatique | Ac | 5-OAc | 6-OAc | H | H |
| $SO_2$ | aromatique | H | 5-OH | 6-OH | H | H |
| O | aromatique | H | 5-OH | H | H | H |

| X | Noyau A | $R_1$ | $R_2$ | $R_3$ | $R_4$ | $R_5$ |
|---|---|---|---|---|---|---|
| O | aromatique | H | 5-OAc | H | H | H |
| O | aromatique | OAc<br>$CO_2CHCH_3$ | 5-CH | H | H | H |
| $SO_2$ | aromatique | OAc<br>$CO_2CHCH_3$ | 5-OH | H | H | H |

10. 5-Acétoxy-12H-benzo(a)phénothiazine.
11. 5-Hydroxy-12-(1-acétoxyéthoxycarbonyl)-12H-benzo(a)phénothiazine.
12. 5-Acétoxy-12H-benzo(a)phénothiazine-7,7-dioxyde.
13. 5-Hydroxy-12H-benzo(a)phénothiazine-7,7-dioxyde.
14. 5-Acétoxy-12-(1-acétoxyéthoxycarbonyl)-12H-benzo(a)phénothiazine.
15. 5-Acétoxy-12H-benzo(a)phénoxazine.
16. 5-Hydroxy-12-(1-acétoxyéthoxycarbonyl)-12H-benzo(a)phénoxazine.
17. Composition selon l'une quelconque des revendications 1 à 7, qui contient une quantité efficace d'un second principe actif supplémentaire qui est un médicament anti-inflammatoire non stéroïdien; un agent analgésique périphérique; un inhibiteur de la cyclo-oxygénase; un antagoniste des leucotriènes; un inhibiteur de leucotriènes; un antagoniste des récepteurs $H_2$; un agent antihistaminique; un antagoniste des prostaglandines ou un antagoniste de la thromboxane.
18. Composition selon la revendication 17, dans laquelle le second principe actif est un médicament anti-inflammatoire non stéroïdien.
19. Composition selon la revendication 18, dans laquelle le médicament anti-inflammatoire non stéroidien est l'indométhacine.
20. Composition selon l'une quelconque des revendications 17 à 19, dans laquelle le rapport pondéral du composé défini dans la revendication 1 au second principe actif est compris dans l'intervalle allant de 1000:1 à 1:1000.
21. Composition selon la revendication 20, dans laquelle ledit rapport est compris dans l'intervalle allant de 200:1 à 1: 200.